# EUROPEAN PATENT APPLICATION

(11) **EP 4 674 388 A2**
(43) Date of publication of application: **07.01.2026**
(21) Application number: 25216644.2
(22) Date of filing: 18.01.2023
(51) Int. Cl.: A61F 2/38

(54) **METHODOLOGY FOR UNDERSTANDING KNEE ANATOMY AND DESIGN OF AN ANATOMIC SYSTEM FOR REVISION KNEE ARTHROPLASTY**

(30) Priority: 18.01.2022 US 202263300412 P; 15.04.2022 US 202263331329 P
(62) Divisional of application: 23705191.7
(71) Applicant: Mayo Foundation for Medical Education and Research, Rochester, MN 55905 (US)
(72) Inventor: SPERLING, John W., Rochester, 55902 (US); MABRY, Tad M., Rochester, 55902-1413 (US)
(74) Representative: Samson & Partner Patentanwälte mbB

(57) **Abstract**

A prosthetic system is disclosed for providing motion between a first bone and a second bone of a joint. The system can comprise a support structure (e.g., sleeve or a cone) having a first end surface, a second end surface, an exterior surface extending from the first end surface to the second end surface, and an inner surface defining a passageway extending from the first end surface to the second end surface, wherein the exterior surface of the support structure is configured to be received in a cavity of the first bone such that the first end surface is flush with or beneath a surface of the first bone. The first end surface is within an axial plane defined by the first end surface and an outermost edge of the first end surface, and a longitudinal axis of the passageway of the support structure is offset with respect to a geometric center point of the axial plane.

## Description

### CROSS-REFERENCES TO RELATED APPLICATIONS

This application is based on, claims benefit of, and claims priority to U.S. Application No. 63/300,412 filed on January 18, 2022, and U.S. Application No. 63/331,329 filed on April 15, 2022, which are both hereby incorporated by reference herein in their entirety for all purposes.

### STATEMENT REGARDING FEDERALLY SPONSORED RESEARCH

Not Applicable.

### BACKGROUND OF THE INVENTION

### 1. Field of the Invention

This invention relates to revision total knee arthroplasty components that maximize contact of the component(s) with native bone and minimize the risk of intra-operative fractures.

### 2. Description of the Related Art

In total knee arthroplasty, the articulating elements within the knee joint are replaced. During the surgery, the distal end of the femur is cut to a particular shape, and then a femoral implant is attached. The femoral implant typically includes a pair of convex condylar surfaces which can be attached to a femoral stem for additional fixation within the bone. The proximal end of the tibia is also cut to a particular size or shape, and then the tibial stem is secured in the proximal end of the tibia. Tibial stem extensions may be utilized if needed for additional fixation. The femoral condylar surfaces are shaped to slide within corresponding concave bearing surfaces on a tibial bearing. The tibial bearing is typically formed from the polymeric material such as polyethylene, which allows the femoral condylar surfaces to slide in the concave bearing surfaces with reduced friction. The tibial bearing is attached to the proximal side of a tibial platform.

In some surgical cases, there has been a loss of bone at the distal portion of the femur and/or the proximal portion of the tibia. Multiple techniques have been developed over time in order to compensate for the missing bone, including the use of metaphyseal sleeve augments. Metaphyseal sleeves are attached to the femoral and/or tibial implants around the stem extension(s), and contribute to both early- and long-term implant fixation to bone. A metaphyseal cone, on the other hand, includes where the cone is first placed into the bone, and the femoral and/or tibial component is placed independently. Both metaphyseal sleeves and metaphyseal cones have proven very effective in the management of such bone defects. Furthermore, these devices have been proven to enhance long-term implant fixation.

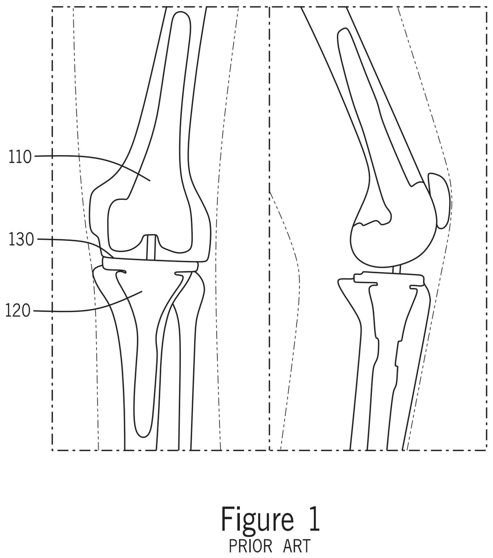

There has been a dramatic growth in primary and revision knee arthroplasty worldwide. By 2030, total knee replacements are projected to grow 673% to 3.5 million procedures annually with the number of revision knee replacements projected to exceed 250,000 in the United States alone. One of the central challenges of revision knee surgery is managing the resultant bone loss inherent to failed prior attempts at knee replacement. Looking at existing devices in the marketplace, there are significant deficiencies. Specifically, current knee revision metaphyseal sleeves 110, 120 are not in an anatomic shape or optimized size distribution. See Figure 1. Additionally, revision tibial and femoral stems are not always properly positioned relative to the sleeves to engage the central aspect of the diaphyseal canal. This can result in implant malalignment in the axial, sagittal, and coronal planes, negatively impacting patient outcome.

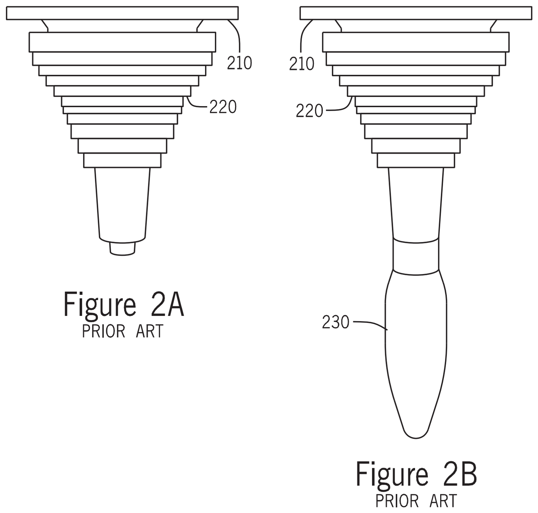

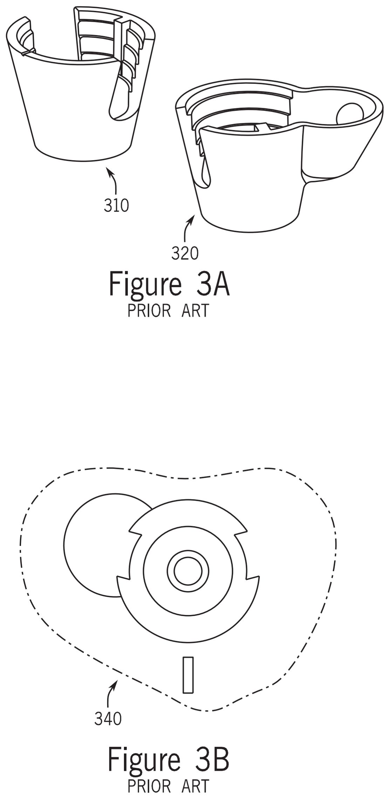

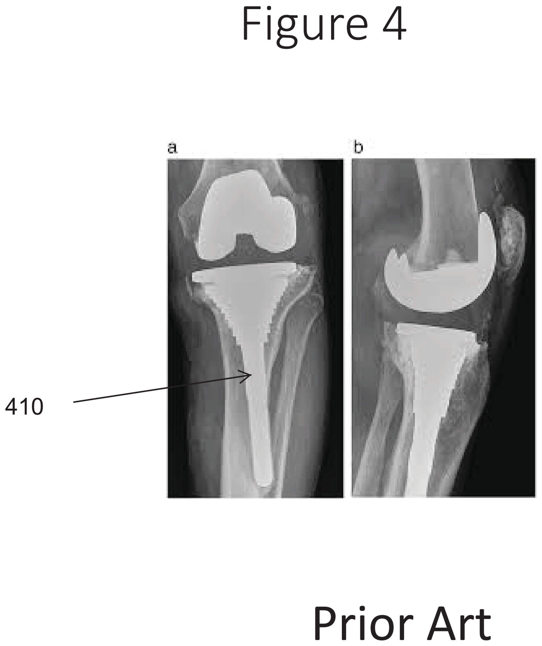

Examination reveals that all of the currently available metaphyseal sleeves on the market used to manage bone deficiency at the time of revision knee replacement have a uniform circular or semi-circular shape and fail to optimally match the underlying anatomy. See Figures 2A and 2B showing the tibial trays 210, sleeves 220, and stem 230. The available sleeves, and similarly available cones, do not take into consideration specific differences between the medial and lateral aspects of the proximal tibia and distal femur. See the cones 310, 320, and 340 in Figures 3A and 3B. The less contact that exists with underlying bone results in higher stress at the device bone interface. This has been associated with early catastrophic loosening as well as lack of long term bone ingrowth. In an effort of surgeons to maximize contact with native bone, there is also a growing tendency for intra-operative fractures due to surgeons forcing a non-anatomic device into an anatomic space or removing an excessive amount of bone to make the implant fit the patient's bone. Surgeons are required to force the anatomy to match the implant rather than having the implant match the anatomy. See the tibial component 410 in Figure 4.

In order to have an implant that maximizes contact with the best quality bone, one needs an implant that matches the proximal tibial and distal femoral anatomy. In addition, the distribution of implant sizes available should be based on a true anatomic distribution as well as side-specific implants.

The need for revision total knee arthroplasty is expected to continue rising dramatically over the coming decades. At the time of revision, the surgeon must achieve durable fixation and ligamentous stability, often in the setting of pre-existing bone loss and ligamentous compromise. Failure to achieve these goals perpetuates the cycle of implant failure and re-revision.

Contemporary revision surgery emphasizes the concept of zonal fixation, with a goal of solid fixation at the joint level, in the metaphysis (sleeves or cones), and in the diaphysis (cemented or cementless stems). When attempting to optimize zonal fixation, there is a breadth of complications that can occur when using implants that are not anatomically correct. These include fracturing the tibia and/or femur during implant preparation or implantation, catastrophic early component loosening when contact with native bone is not optimized, as well as lack of durable fixation (e.g., bone ingrowth into metaphyseal sleeves or cones) leading to late failure.

Therefore, there exists an unmet need for revision joint arthroplasty components that maximize contact of the component(s) with native bone and minimize the risk of intra-operative fractures.

### SUMMARY OF THE INVENTION

The notable limitations of current revision knee components resulted in a comprehensive investigation into the cause of these problems and the resultant novel methodology that addresses them.

The current method maximizes device contact with native bone and minimizes risk of fracture. In the past, some sleeves and cones have been used in a cemented manner due to poor fit with underlying bone. Advantages of improved uncemented revision knee components would include more durable long-term implant fixation, shorter operating room time, avoidance of morbidity associated with the use of cement, and the potential for less-invasive future revision surgery if needed. The methodology results in an intuitive, streamlined workflow that can greatly improve the surgeon experience and improve patient care.

Disclosed herein is a methodology resulting in a deep understanding of the anatomy and developed intuitive instrumentation and implants that can greatly facilitate revision knee arthroplasty through the use of CT scan data and 3D modeling. This methodology describes the interaction of anatomical features of the proximal tibia and distal femur and how these features change based on the specific location in the bone. Additionally, the methodology has demonstrated that the shape of the proximal tibia and distal femoral regions are quite side-specific. Therefore, having right- and left- specific revision implants with an anatomic shape in a true population-based distribution can optimize loading and fit at the bone-device interface, allowing for optimal implant contact against viable host bone, and thereby maximizing long-term fixation.

The invention provides significant insight into the underlying anatomy and clearly characterizes that the proximal tibial and distal femoral anatomy is not uniform. Based on the specific location within the proximal tibia and distal femur, the underlying architecture and three-dimensional structure changes significantly. This difference has not been adequately taken into consideration with existing revision total knee arthroplasty devices.

In one aspect, the present disclosure provides a prosthetic system for providing motion between a first bone and a second bone of a joint. The system can comprise a support structure having a first end surface, a second end surface, an exterior surface extending from the first end surface to the second end surface, and an inner surface defining a passageway extending from the first end surface to the second end surface, wherein the exterior surface of the support structure is configured to be received in a cavity of the first bone such that the first end surface is flush with or beneath a surface of the first bone, wherein the first end surface is within an axial plane defined by the first end surface and an outermost edge of the first end surface, and wherein a longitudinal axis of the passageway of the support structure is offset with respect to a geometric center point of the axial plane.

In one embodiment of the prosthetic system, the first bone is the tibia, and the joint is the knee. This prosthetic system may further comprise a tibial implant having a body and a stem extending away from the body, wherein the stem is positioned within the passageway of the support structure. The body of the tibial implant can be a tibial tray. The prosthetic system can further comprise a tibial bearing in contact with the tibial tray, the tibial bearing having a bearing surface for articulating with the articulating surfaces of a femoral component.

In one embodiment of the prosthetic system, the first bone is the femur, and the joint is the knee. This prosthetic system can further comprise a femoral component having medial and lateral condyles with curved articulating surfaces. This prosthetic system can further comprise a tibial tray in contact with a tibial bearing having a bearing surface for articulating with the articulating surfaces of the femoral component.

In one embodiment of the prosthetic system, the longitudinal axis of the passageway of the support structure is angled at an oblique angle with respect to a normal line to the axial plane. The oblique angle can be greater than 0 degrees and less than 10 degrees. The oblique angle can be greater than 3 degrees and less than 9 degrees.

In one embodiment of the prosthetic system, the longitudinal axis of the passageway of the support structure is an intramedullary axis including a first intersection point and a second intersection point, the first intersection point being defined by a first intersection of an inertial axis of the first bone with a first reference axial plane located at a first distance from an end surface of the first bone before resection, and the second intersection point being defined by a second intersection of the inertial axis of the first bone with a second reference axial plane located at a second distance from the end surface of the first bone before resection, the first distance and the second distance being different. The first distance can be between 25 millimeters and 125 millimeters, and the second distance can be between 75 millimeters and 200 millimeters.

In one embodiment, the prosthetic system can further comprise a prosthetic implant having a body and a stem extending away from the body, wherein the stem is positioned within the passageway of the support structure. In one embodiment of the prosthetic system, the support structure can be a sleeve, and the sleeve can be attached to the stem of the prosthetic implant. In one embodiment of the prosthetic system, the support structure can be a cone.

In one embodiment of the prosthetic system, the exterior surface of the support structure is stepped. In another embodiment of the prosthetic system, the exterior surface of the support structure is smooth. In another embodiment of the prosthetic system, the exterior surface of the support structure is roughened. In another embodiment of the prosthetic system, the exterior surface of the support structure comprises a porous ingrowth material.

In one embodiment of the prosthetic system, the support structure has a wall between the exterior surface and the inner surface, and the wall includes one or more notches extending away from the first end surface. In one embodiment of the prosthetic system, the wall is an anterior wall. In another embodiment of the prosthetic system, the wall is a posterior wall.

In one embodiment of the prosthetic system, the support structure has a wall between the exterior surface and the inner surface, and the wall includes one or more notches extending away from the second end surface. In one embodiment, the wall is an anterior wall. In another embodiment, the wall is a posterior wall.

In one embodiment of the prosthetic system, the support structure has a wall between the exterior surface and the inner surface, and an anterior section of the wall has reduced thickness compared to another section of the wall adjacent to the anterior section of the wall. In another embodiment of the prosthetic system, the outermost edge of the first end surface is a perimeter of the first end surface. In another embodiment of the prosthetic system, the first end surface includes one or more slots extending from the passageway, and each slot is dimensioned to receive a stabilization arm of a stem of a prosthetic implant.

In another aspect, the present disclosure provides a kit for a prosthetic system for providing motion between a first bone and a second bone of a joint. The kit can include (i) a first support structure having a first end surface, a second end surface, an exterior surface extending from the first end surface to the second end surface, and an inner surface defining a passageway extending from the first end surface to the second end surface, wherein the exterior surface of the first support structure is configured to be received in a cavity of the first bone such that the first end surface is flush with or beneath a surface of the first bone, wherein the first end surface is within an axial plane defined by the first end surface and an outermost edge of the first end surface, and wherein a longitudinal axis of the passageway of the first support structure is offset with respect to a geometric center point of the axial plane; and (ii) one or more additional support structures, each additional support structure having an end surface within an additional axial plane defined by the end surface of each additional support structure and a border of the end surface of each additional support structure, wherein each longitudinal axis of a passageway extending through each additional support structure is offset with respect to a geometric center point of the additional axial plane.

In one embodiment of the kit, the first bone is the tibia, the joint is the knee, and the first support structure and each additional support structure are configured to be received in a cavity of the tibia.

In one embodiment of the kit, the first bone is the femur, the joint is the knee, and the first support structure and each additional support structure are configured to be received in a cavity of the femur.

In one embodiment of the kit, the longitudinal axis of the passageway of the first support structure is angled at an oblique angle with respect to a normal line to the axial plane. The oblique angle can be greater than 0 degrees and less than 10 degrees. The oblique angle can be greater than 3 degrees and less than 9 degrees.

In one embodiment of the kit, the longitudinal axis of the passageway of the first support structure is an intramedullary axis including a first intersection point and a second intersection point, the first intersection point being defined by a first intersection of an inertial axis of the first bone with a first reference axial plane located at a first distance from an end surface of the first bone before resection, and the second intersection point being defined by a second intersection of the inertial axis of the first bone with a second reference axial plane located at a second distance from the end surface of the first bone before resection, the first distance and the second distance being different.

In one embodiment of the kit, the first support structure and each additional support structure are a sleeve, and each sleeve is dimensioned to be attached to a stem of a prosthetic implant. In another embodiment of the kit, the first support structure and each additional support structure are a cone.

In one embodiment of the kit, the exterior surface of the first support structure is stepped. In one embodiment of the kit, the exterior surface of the first support structure is smooth. In one embodiment of the kit, the exterior surface of the support structure is roughened. In one embodiment of the kit, the exterior surface of the support structure comprises a porous ingrowth material.

In one embodiment of the kit, the first support structure has a wall between the exterior surface and the inner surface, and the wall includes one or more notches extending away from the first end surface. In one embodiment, the wall is an anterior wall. In another embodiment, the wall is a posterior wall.

In one embodiment of the kit, the first support structure has a wall between the exterior surface and the inner surface, and the wall includes one or more notches extending away from the second end surface. In one embodiment, the wall is an anterior wall. In another embodiment, the wall is a posterior wall.

In one embodiment of the kit, the first support structure has a wall between the exterior surface and the inner surface, and an anterior section of the wall has reduced thickness compared to another section of the wall adjacent to the anterior section of the wall. In one embodiment of the kit, the outermost edge of the first end surface is a perimeter of the first end surface. In one embodiment of the kit, the first end surface includes one or more slots extending from the passageway, and each slot is dimensioned to receive a stabilization arm of a stem of a prosthetic implant.

In yet another aspect, the present disclosure provides a method for manufacturing a prosthetic support structure for implantation in a cavity in an end of a bone. The method comprises forming a prosthetic support structure having an exterior surface dimensioned to fit in the cavity and having an inner surface which defines a passageway of the support structure, the passageway being dimensioned to receive a stem of a prosthetic implant, the passageway extending from a first end surface to a second end surface of the support structure, the first end surface of the support structure being within an axial plane defined by the first end surface and an outermost edge of the first end surface, the passageway having a longitudinal axis, an intersection point between the axial plane and the longitudinal axis of the passageway having been determined by: (i) obtaining an image of a reference bone, (ii) orienting on the image a first reference axial plane located at a first distance from an end surface of the reference bone, (iii) orienting on the image a second reference axial plane located at a second distance from the end surface of the reference bone, the first distance and the second distance being different, (iv) orienting on the image an intramedullary axis by connecting a first intersection point and a second intersection point, the first intersection point being defined by a first intersection of an inertial axis of the reference bone with the first reference axial plane, the second intersection point being defined by a second intersection of the inertial axis of the reference bone with the second reference axial plane, (v) orienting on the image a reference resection plane having a border defined by an axial image of the reference bone, and (vi) orienting on the image a reference intersection point between the reference resection plane and the intramedullary axis, wherein a reference spatial relationship between the reference intersection point and the border of the reference resection plane corresponds to a spatial relationship of the intersection point on the axial plane and the outermost edge of the first end surface of the support structure.

In one embodiment of the method, the longitudinal axis of the passageway of the support structure is offset with respect to a geometric center point of the axial plane.

In one embodiment of the method, the bone is the tibia. In one embodiment of the method, the bone is the femur.

In one embodiment of the method, the longitudinal axis of the passageway of the support structure is angled at an oblique angle with respect to a normal line to the axial plane. The oblique angle can be greater than 0 degrees and less than 10 degrees. The oblique angle can be greater than 3 degrees and less than 9 degrees.

In one embodiment of the method, the first distance is between 25 millimeters and 125 millimeters, and the second distance is between 75 millimeters and 200 millimeters.

The method can further comprise the spatial relationship of the intersection point on the axial plane and the outermost edge of the first end surface of the support structure having been determined by: orienting radial measurements on an axial image of the reference bone, the radial measurements being between the reference intersection point and the border of the reference resection plane.

The method can further comprise the spatial relationship of the intersection point on the axial plane and the outermost edge of the first end surface of the support structure having been determined by: orienting an angle measurement on an axial image of the reference bone, the angle measurement being between the reference intersection point and a geometric center point of the reference resection plane.

The method can further comprise the spatial relationship of the intersection point on the axial plane and the outermost edge of the first end surface of the support structure having been determined by: orienting a medial-lateral length measurement on an axial image of the reference bone, the medial-lateral length measurement being between medial and lateral sides of the border of the reference resection plane.

In one embodiment of the method, the support structure is a sleeve, and the sleeve is configured to attach to the stem of the prosthetic implant. In one embodiment of the method, the support structure is a cone.

Advantages of prosthetic systems, kits, and methods of the present disclosure include, without limitation, the following:
∘ offset of the stem inside of the sleeve / cone allows for more anatomic positioning;
o anatomic shape and wider distribution of sizes for the metaphyseal sleeve / cone enhances: (i) simplicity of technique, avoids rotational / translational / size compromises, (ii) applicability to a wider range of anatomy, and (iii) flexibility to choose whether to use a sleeve vs. cone construct with same instruments;
∘ the specifics of smooth-walled vs. stepped-walled, and specific dimensions can be flexible, based on needs and wishes of commercial partner, e.g., a company might want to build only four sleeves instead of six sleeves, and re-distribute the specific final sizes within the range of prosthetic systems and kits of the present disclosure;
∘ the specific interior dimensions of sleeves and cones of the present disclosure are flexible to allow any individual commercial partner to customize to their revision system; and
∘ the ability to have a customizable interior within the exterior shapes defined by sleeves and cones of the present disclosure.

Advantageous features of sleeves and cones of the present disclosure include, without limitation:
∘ the exterior shape design and stem offset;
∘ the interior dimensions can be customized to the specific implant design from a commercial partner; and
∘ the ability to make a smooth-walled exterior or a stepped-walled model which can make it easier to make the instruments and implants, and facilitate applications for robotic-assisted preparation for the sleeve / cone.

These and other features, aspects, and advantages of the present invention will become better understood upon consideration of the following detailed description, drawings and appended claims.

### BRIEF DESCRIPTION OF THE DRAWINGS

Figure 1 shows anterior and lateral X-ray images of a prior art revision knee prosthesis system having a femoral component including a femoral augment in the form of a sleeve and a tibial component including a tibial augment in the form of a sleeve.
Figure 2A shows an anterior view of a prior art tibial component including a tibial augment in the form of a sleeve.
Figure 2B shows another anterior view of a prior art tibial component including a tibial augment in the form of a sleeve.
Figure 3A shows perspective views of prior art tibial augments in the form of cones.
Figure 3B shows a top view of a prior art tibial augment in the form of a cone implanted in the proximal end of the tibia.
Figure 4 shows anterior (panel a) and lateral (panel b) X-ray images of another prior art revision knee prosthesis system having a femoral component and a malaligned tibial component including a tibial augment in the form of a sleeve.
Figure 5 shows an anterior coronal image of a tibia with measurement lines according to the invention to cancellous bone at the points on planes 50 millimeters (mm.) and 100 millimeters distal to the tibial lateral cortical plateau.
Figure 6 shows an anterior coronal image of a tibia with a reference tibia resection plane showing the position of a standard tibia cut for creating a tibial resection surface for total knee arthroplasty.
Figure 7A shows an axial slice of an image of the tibia with measurement lines according to the invention wherein the geometric center point is defined as the centroid of the slice and the distance from the intramedullary (IM) axis point to the geometric center point is measured.
Figure 7B shows an anterior coronal image of a tibia with measurement lines according to the invention at axial slices spaced 5 millimeters apart from the reference tibia resection plane as shown in Figure 6 to a distance 50 millimeters distal to the reference tibia resection plane wherein the geometric center points as in Figure 7A are on the right side of Figure 7B and the intramedullary axis points as in Figure 7A are on the left side of Figure 7B.
Figure 8 shows an axial slice of an image of the tibia with measurement lines according to the invention wherein the angle deviation is a measurement of the angle between the medial-lateral axis and a line intersecting the intramedullary axis point as in Figure 7A and the geometric center point as in Figure 7A.
Figure 9 shows an axial slice of an image of the tibia with measurement lines according to the invention through the geometric center point of anterior-posterior width and medial-lateral width.

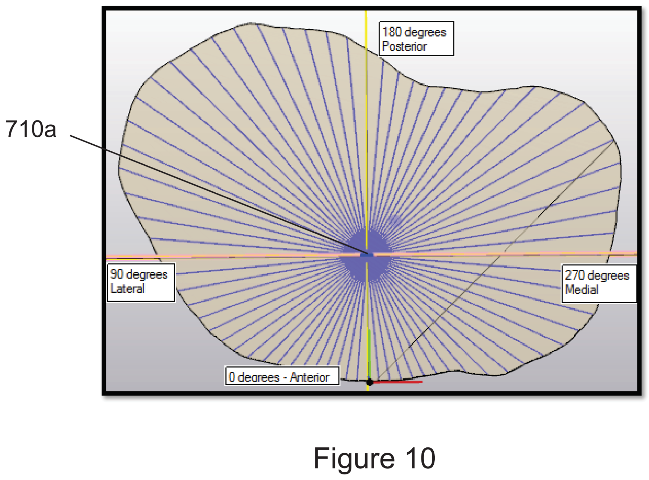

Figure 10 shows an axial slice of an image of the tibia with measurement lines according to the invention wherein radial measurements are based around the intramedullary axis point as in Figure 7A of the slice and a distance is measured from this intramedullary axis point to the edge of the tibia of the slice every 5 degrees clockwise, anterior, lateral, posterior, and medial, respectively.
Figure 11 shows a table with the maximum medial-lateral width of the reference tibia resection plane as shown in Figure 5 wherein the proximal tibia is placed into groups every 4 millimeters. The tibias were grouped by width: 54-58 mm., 58-62 mm., 62-66 mm., 66-70 mm., 70-74 mm., 74-78 mm., and 78-82 mm.
Figure 12A is a graph showing the total distance between the intramedullary axis point as in Figure 7 and the geometric center point as in Figure 7 moving proximal to distal down the tibia at 10 mm. increments from the reference tibia resection plane as shown in Figure 5 for all groups of Figure 11.
Figure 12B is a graph showing the total distance between the intramedullary axis point as in Figure 7 and the geometric center point as in Figure 7 moving proximal to distal down the tibia at 10 mm. increments from the reference tibia resection plane as shown in Figure 5 for a total average.
Figure 13A is a graph showing the average deviation between the intramedullary axis point as in Figure 7 and the geometric center point as in Figure 7 moving proximal to distal down the tibia at 10 mm. increments from the reference tibia resection plane as shown in Figure 5 for all groups of Figure 11.
Figure 13B is a graph showing the average deviation between the intramedullary axis point as in Figure 7 and the geometric center point as in Figure 7 moving proximal to distal down the tibia at 10 mm. increments from the reference tibia resection plane as shown in Figure 5 for a total average.
Figure 14A is a graph showing the angle deviation of the angle between the medial-lateral axis and a line intersecting the intramedullary axis point as in Figure 7 and the geometric center point as in Figure 7 moving proximal to distal down the tibia at 5 mm. increments up to 50 mm. from the reference tibia resection plane as shown in Figure 5 for all groups of Figure 11.
Figure 14B is a graph showing the angle deviation of the angle between the medial-lateral axis and a line intersecting the intramedullary axis point as in Figure 7 and the geometric center point as in Figure 7 moving proximal to distal down the tibia at 5 mm. increments up to 50 mm. from the reference tibia resection plane as shown in Figure 5.
Figure 15 is a graph showing radial measurements as in Figure 10 for each of the groups of Figure 11.
Figure 16 shows a medial sagittal image of a femur with measurement lines according to the invention to cancellous bone at the points 75 millimeters (mm.) and 175 millimeters proximal to the distal end surface of the medial side of the femur.
Figure 17 shows an anterior coronal view of an image of a femur with measurement lines according to the invention for medial-lateral width.
Figure 18 shows an axial slice of an image of the femur with measurement lines according to the invention wherein the geometric center point is defined as the centroid of the slice and the distance from the intramedullary (IM) axis point to the geometric center point is measured.
Figure 19 shows a lateral sagittal image of the femur with axial slices based around the intramedullary axis point as in Figure 16.
Figure 20A shows an axial slice of an image of the femur with measurement lines according to the invention wherein radial measurements are based around the intramedullary axis point as in Figure 18 of the slice and a distance is measured from this intramedullary axis point to the edge of the femur of the slice every 5 degrees clockwise, anterior, lateral, posterior, and medial, respectively.
Figure 20B shows an anterior coronal view of an image of a femur with a reference femur resection plane showing the position of a standard femoral cut for creating a femoral resection surface for total knee arthroplasty.
Figure 21 shows a table with the maximum medial-lateral width of the reference femur resection plane as shown in Figure 16 wherein the distal femur is placed into groups every 5 millimeters. The femurs were grouped by width: 65-70 mm., 71-75 mm., 75-80 mm., 80-85 mm., and 85+ mm.
Figure 22 is a graph showing the total distance between the intramedullary axis point as in Figure 18 and the geometric center point as in Figure 18 moving distal to proximal up the femur at 10 mm. increments from the reference femur resection plane as shown in Figure 16 for all groups of Figure 21.
Figure 23 is a graph showing radial measurements as in Figure 20 for each of the groups of Figure 21.
Figure 24A is a side exploded view of a tibial component having a sleeve according to one example embodiment of the present disclosure.
Figure 24B is another side exploded view of the tibial component of Figure 24A.
Figure 24C is a perspective view of components of an example embodiment of an anatomically shaped tibial component having a sleeve according to the present disclosure.
Figure 25A shows a side view of a femoral component having a sleeve (left) and a proximal view of various anatomically shaped femoral sleeves according to the present disclosure (right).
Figure 25B shows perspective views of various anatomically shaped femoral sleeves according to the present disclosure.
Figure 25C shows perspective views of various anatomically shaped femoral sleeves according to the present disclosure.
Figure 26 shows typical femoral and tibial bone defects in revision knee arthroplasty.
Figure 27A shows a step in a tibia preparation technique to ream a distal tibia to cortical contact.
Figure 27B shows a step in a tibia preparation technique to tibial broach and trial a stem.
Figure 27C shows a step in a tibia preparation technique to impact broach and trial a stem.
Figure 28A is a top view of a sleeve in a proximal tibia showing how the sleeve according to the present disclosure matches the proximal tibial anatomy and creates an optimized environment for stability and bone ingrowth.
Figure 28B is a top view of a proximal tibia showing how the proximal tibial anatomy is prepared to create an optimized environment for stability and bone ingrowth of a sleeve.
Figure 29A shows a step in the impaction of a tibial sleeve and stem construct according to the present disclosure.
Figure 29B shows a top view of a sleeve in a proximal tibia showing how the sleeve according to the present disclosure matches the proximal tibial anatomy and creates an optimized environment for stability and bone ingrowth.
Figure 30A shows a step in a femur preparation technique to ream femur to cortical contact.
Figure 30B shows a step in a femur preparation technique to femoral broach and trial stem.
Figure 31A is a side view of a sleeve according to the present disclosure that matches the distal femoral anatomy and creates an optimized environment for stability and bone ingrowth.
Figure 31B is a bottom view of a distal femur showing how the distal femur anatomy is prepared to create an optimized environment for stability and bone ingrowth of a sleeve.
Figure 32A is a perspective view of a tibial sleeve according to the invention wherein the opening in the sleeve for the stem is offset in an anterior and medial direction relative to the geometric center point of the cancellous bone at the proximal aspect of the tibia.
Figure 32B is an anterior view of the tibial sleeve of Figure 32A wherein the opening in the sleeve for the stem is in line with the canal of the tibia.
Figure 33A shows top views of tibial sleeves according to the invention wherein the sleeves grow incrementally greater medially compared to laterally which is consistent with the underlying anatomy.
Figure 33B shows anterior views that correspond left to right with the left to right tibial sleeves of Figure 33A wherein the tibial sleeves increase in size by adding an incremental step which makes moving up and down sizes intuitive and the instrumentation streamlined.
Figure 34A is an anterior view of a tibial sleeve according to the invention wherein the tibial sleeve design matches the underlying anatomy preserving bone and optimizing implant-bone surface area contact for long term ingrowth.
Figure 34B is a top perspective view of the tibial sleeve of Figure 34A.
Figure 34C shows a top view of an embodiment of a tibial sleeve according to the invention.
Figure 34D shows an anterior view of the tibial sleeve of Figure 34C.
Figure 35A is a bottom view of a femoral sleeve according to the invention wherein the femoral sleeve design matches the underlying anatomy preserving bone and optimizing the implant.
Figure 35B is an anterior view of a femoral sleeve of Figure 35A.
Figure 36A shows top views of femoral sleeves according to the invention wherein the opening in the femoral sleeve for the stem has a 6 degree angle corresponding to the canal of the femur.
Figure 36B shows anterior views that correspond left to right with the left to right femoral sleeves of Figure 36A wherein the femoral sleeves increase in size by adding an incremental step which makes moving up and down sizes intuitive and the instrumentation streamlined.
Figure 36C shows a top view of an embodiment of a femoral sleeve according to the invention.
Figure 36D shows an anterior view of the femoral sleeve of Figure 36C.
Figure 37A is a perspective view of a tibial cone according to the invention wherein the opening in the cone for the stem is offset in an anterior and medial direction relative to the geometric center point of the cancellous bone at the proximal aspect of the tibia.
Figure 37B is an anterior view of the tibial cone of Figure 37A wherein the opening in the cone for the stem is in line with the canal of the tibia.
Figure 38A shows top views of tibial cones according to the invention wherein the cones grow incrementally greater medially compared to laterally which is consistent with the underlying anatomy.
Figure 38B shows anterior views that correspond left to right with the left to right tibial cones of Figure 38A wherein the tibial cones increase in size by adding an incremental step which makes moving up and down sizes intuitive and the instrumentation streamlined.
Figure 39A is an anterior view of a tibial cone according to the invention wherein the tibial cone design matches the underlying anatomy preserving bone and optimizing implant-bone surface area contact for long term ingrowth.
Figure 39B is a top perspective view of the tibial cone of Figure 39A.
Figure 39C shows a top view of an embodiment of a tibial cone according to the invention.
Figure 39D shows an anterior view of the tibial cone of Figure 39C.
Figure 40A is a bottom view of a femoral cone according to the invention wherein the femoral cone design matches the underlying anatomy preserving bone and optimizing the implant.
Figure 40B shows is an anterior view of a femoral cone of Figure 40A.
Figure 41A shows top views of femoral cones according to the invention wherein the opening in the femoral cone for the stem has a 6 degree angle corresponding to the canal of the femur.
Figure 41B shows anterior views that correspond left to right with the left to right femoral cones of Figure 41A wherein the femoral cones increase in size by adding an incremental step which makes moving up and down sizes intuitive and the instrumentation streamlined.
Figure 41C shows a top view of an embodiment of a femoral cone according to the invention.
Figure 41D shows an anterior view of the femoral cone of Figure 41C.
Figure 41E shows a top view of another embodiment of a femoral cone according to the invention having a portion of the anterior wall removed.
Figure 41F shows an anterior view of the femoral cone of Figure 41E.
Figure 41G shows a top view of yet another embodiment of a femoral cone according to the invention wherein the anterior wall of the femoral cone is thinner to accommodate the bow of the femur which increases sagittal plane freedom when placing a femoral stem.
Figure 42 shows a top anterior perspective view of a non-limiting example tibial cone with a portion of the wall removed to accommodate a large size stem or for placing the stem in a range of positions and angulations.
Figure 43 shows top anterior perspective views that correspond to the tibial cone of Figure 42 wherein the tibial cones increase in size by adding an incremental step which makes moving up and down sizes intuitive and the instrumentation streamlined.
Figure 44 shows a top anterior perspective view of another non-limiting example tibial cone with a stepped outer wall and notches for tibial component fins.
Figure 45 shows a top anterior perspective view of yet another non-limiting example tibial cone with a smooth outer wall and notches for tibial component fins.

Like reference numerals will be used to refer to like parts from Figure to Figure in the following description of the drawings.

### DETAILED DESCRIPTION OF THE INVENTION

Before the present invention is described in further detail, it is to be understood that the invention is not limited to the particular embodiments described. It is also to be understood that the terminology used herein is for the purpose of describing particular embodiments only, and is not intended to be limiting. The scope of the present invention will be limited only by the claims. As used herein, the singular forms "a", "an", and "the" include plural embodiments unless the context clearly dictates otherwise.

It should be apparent to those skilled in the art that many additional modifications beside those described herein are possible without departing from the inventive concepts. In interpreting this disclosure, all terms should be interpreted in the broadest possible manner consistent with the context. Variations of the term "comprising", "including", or "having" should be interpreted as referring to elements, components, or steps in a non-exclusive manner, so the referenced elements, components, or steps may be combined with other elements, components, or steps that are not expressly referenced. Embodiments referenced as "comprising", "including", or "having" certain elements are also contemplated as "consisting essentially of" and "consisting of" those elements, unless the context clearly dictates otherwise. It should be appreciated that aspects of the disclosure that are described with respect to a device are applicable to the methods, and vice versa, unless the context explicitly dictates otherwise.

In one aspect, the present disclosure provides a prosthetic system for providing motion between a first bone and a second bone of a joint. The system can comprise a support structure (e.g., sleeve or a cone) having a first end surface, a second end surface, an exterior surface extending from the first end surface to the second end surface, and an inner surface defining a passageway extending from the first end surface to the second end surface, wherein the exterior surface of the sleeve is configured to be received in a cavity of the first bone such that the first end surface is flush with or beneath a surface of the first bone. The first end surface is within an axial plane defined by the first end surface and an outermost edge of the first end surface, and a longitudinal axis of the passageway of the support structure (e.g., sleeve or cone) is offset with respect to a geometric center point of the axial plane. In one embodiment, the first bone is the tibia, and the joint is the knee. In another embodiment, the first bone is the femur, and the joint is the knee.

In another aspect, the present disclosure provides a kit for a prosthetic system for providing motion between a first bone and a second bone of a joint. The kit can include a first support structure (e.g., sleeve or cone) according to the present disclosure, and one or more additional support structures (e.g., sleeves or cones) of a different size according to the present disclosure.

In yet another aspect, the present disclosure provides a method for manufacturing a prosthetic support structure (e.g., sleeve or cone) for implantation in a cavity in an end of a bone. The method comprises forming a prosthetic support structure (e.g., sleeve or cone) having an exterior surface dimensioned to fit in the cavity in the end of the bone and having an inner surface which defines a passageway of the support structure (e.g., sleeve or cone). The passageway is dimensioned to receive a stem of a prosthetic implant, and the passageway extends from a first end surface to a second end surface of the support structure (e.g., sleeve or cone). The first end surface of the support structure (e.g., sleeve or cone) is within an axial plane defined by the first end surface and an outermost edge of the first end surface, and the passageway has a longitudinal axis. An intersection point between the axial plane and the longitudinal axis of the passageway has been determined by: (i) obtaining an image of a reference bone, (ii) orienting on the image a first reference axial plane located at a first distance from an end surface of the reference bone, (iii) orienting on the image a second reference axial plane located at a second distance from the end surface of the reference bone, the first distance and the second distance being different, (iv) orienting on the image an intramedullary axis by connecting a first intersection point and a second intersection point, the first intersection point being defined by a first intersection of an inertial axis of the reference bone with the first reference axial plane, the second intersection point being defined by a second intersection of the inertial axis of the reference bone with the second reference axial plane, (v) orienting on the image a reference resection plane having a border defined by an axial image of the reference bone, and (vi) orienting on the image a reference intersection point between the reference resection plane and the intramedullary axis, wherein a reference spatial relationship between the reference intersection point and the border of the reference resection plane corresponds to a spatial relationship of the intersection point on the axial plane and the outermost edge of the first end surface of the support structure (e.g., sleeve or cone). In one embodiment, the bone is the tibia. In another embodiment, the bone is the femur.

### EXAMPLES

The following Examples are provided in order to demonstrate and further illustrate certain embodiments and aspects of the present invention and are not to be construed as limiting the scope of the invention.

### Project Resources

A unique database of 50 consecutive high resolution thin cut two dimensional and three-dimensional computerized tomography (CT) scans with a custom designed bone stock protocol was available for study. This custom designed protocol was specifically developed at the Mayo Clinic, Rochester, Minnesota, USA, for a detailed understanding of the anatomy of patients. In addition, 3D modeling of each of these patients was performed. A novel method for understanding proximal tibia and distal femur anatomy, 3D modeling, was subsequently developed using this unique resource. For CT image measurement and computing, Materialise 3-matic^{®} Medical software interface and image segmentation system was used.

### Method, Results and Implications

### A. Proximal Tibial Anatomy

### 1. Defining the Intramedullary Axis (IM Axis)

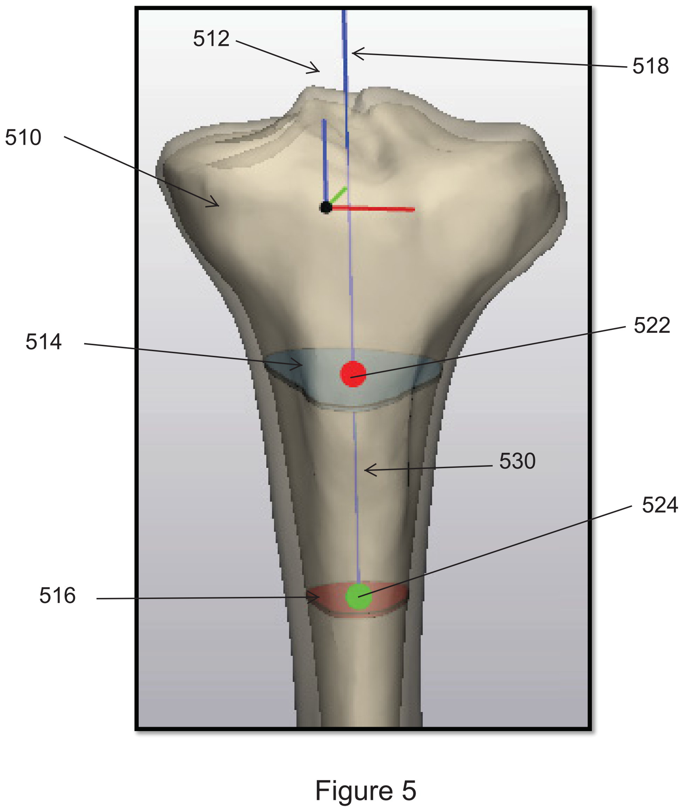

Looking at Figure 5, on the CT image of a tibia 510, points were marked at 50 mm. (5 cm.) and 100 mm. (10 cm.) off of the tibial lateral cortical plateau 512. A first plane 514 and a second plane 516 were created perpendicular to the tibia bone along the axial planes at the 50 mm. and 100 mm. points. The image of cancellous bone with these planes was cut while keeping the original cancellous bone. The top and bottom cancellous parts were hid along with the planes that were just created. An inertial axis line 518 was created based on mesh on the top and bottom surfaces. A first intersection point 522 and a second intersection point 524 of the inertial axis line with the 50 mm. and 100 mm. surfaces respectively were drawn. An intramedullary axis 530 was created by connecting these two points 522, 524.

### 2. Creating the Total Knee Arthroplasty (TKA) Cut

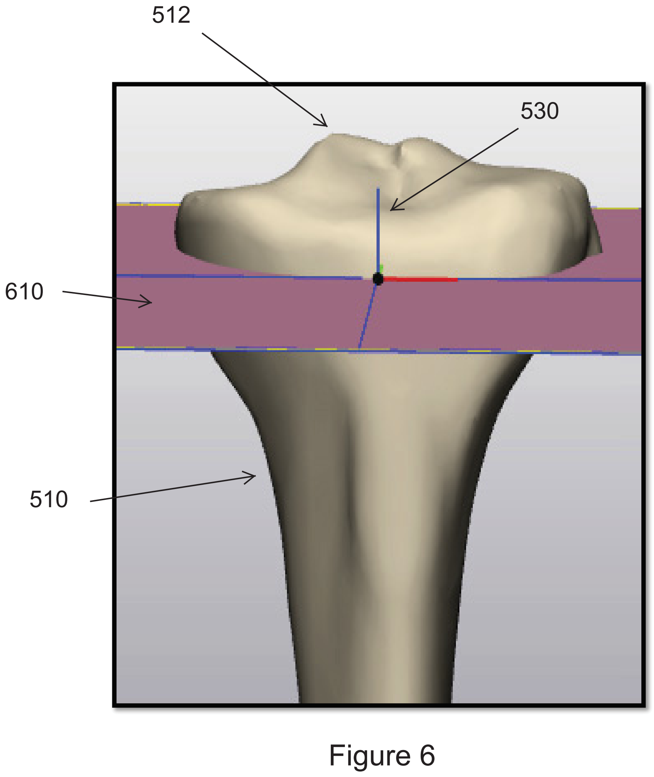

Looking at Figure 6, a reference tibia resection plane 610 corresponding to a standard tibia resection for total knee arthroplasty was drawn on each of the CT tibial scans. The origin of the reference tibia resection plane 610 is created 10 mm. down from the lateral tibia plateau 512 just left of center above the tibial tuberosity. This reference tibia resection plane 610 is placed normal to the intramedullary axis (IM Axis) 530. The plane 610 is rotated in the XY/axial/transverse plane to be aligned with the widest points extending from the medial to lateral plateaus. The plane 610 is rotated down 3 degrees to the medial and then posterior side of the tibia 510.

### 3. Deviation from IM Axis Measurement

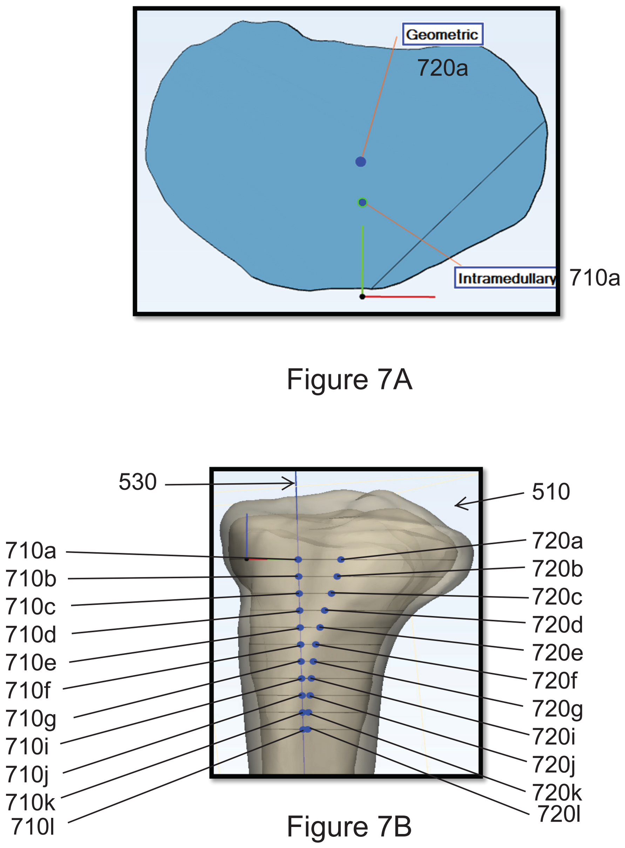

Referring now to Figures 7A and 7B, the intramedullary points 710a to 710l are defined as the point of intersection between each axial slice and the intramedullary axis 530. This is useful as a reference point to make various measurements. The geometric center points 720a to 720l are the centroid of each of the axial slices. The centroid of a plane figure is the arithmetic mean position of all the points in the plane figure. The centroid is the point at which a cutout of the shape could be perfectly balanced on the tip of a pin.

The distance between these two points 710a and 720a represents the offset between the shaft and proximal tibia.

### 4. Angle Deviation Measurement

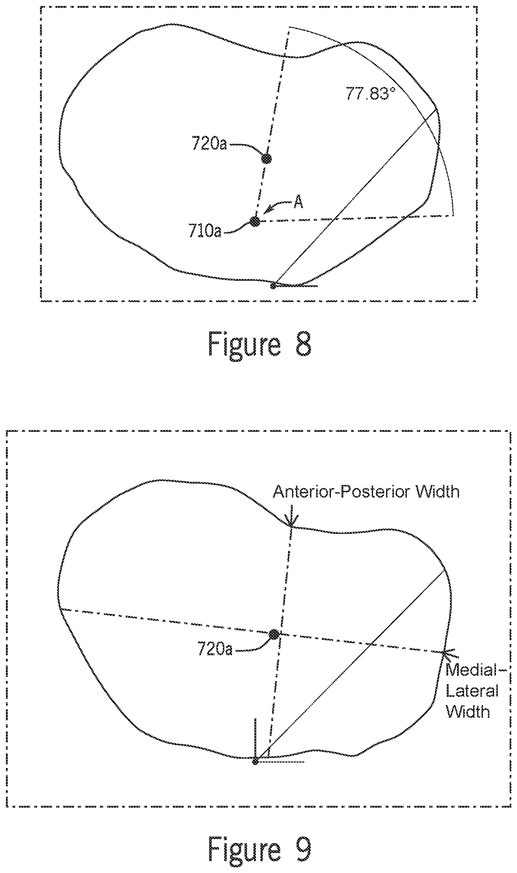

Looking at Figure 8, the angle deviation was a measurement of the angle A (e.g., 77.83° in Figure 8) between the intramedullary axis point 710a, the geometric center point 720a, and the x-axis.

### 5. Total Width Measurement

Looking at Figure 9, on each axial slice, the width was measured in both directions through the geometric center point 720a of the slice. The anterior-posterior width runs from the PCL insertion point to the center of the tibial tuberosity. The medial-lateral width runs perpendicular to the A-P Width encompassing the maximum width of the slice.

### 6. Radial Measurements

Radial measurements were based around the intramedullary center point 710a of the axial slice. A distance is measured from this intramedullary center point 710a to the edge of the slice every 5 degrees clockwise, anterior, lateral, posterior, and then medial, respectively. This data was averaged for each group to create a general outline of the cancellous bone in each axial slice.

### 7. Proximal Tibia Results

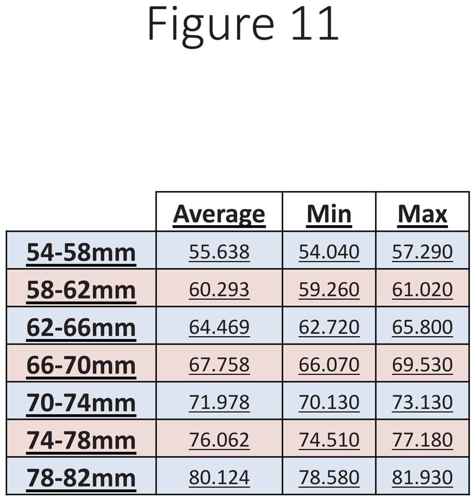

The medial-lateral width of the proximal tibia was placed into groups every 4 millimeters. The tibias were grouped by width: 54-58 mm., 58-62 mm., 62-66 mm., 66-70 mm., 70-74 mm., 74-78 mm., and 78-82 mm. See Figure 11.

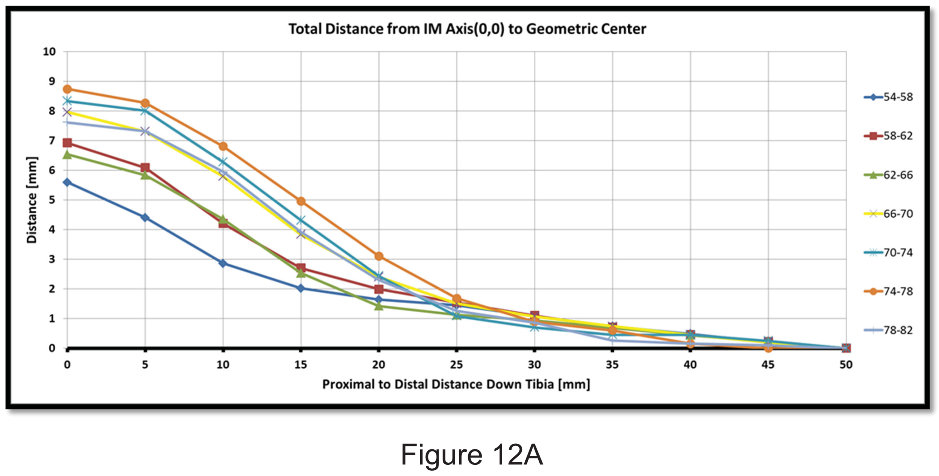

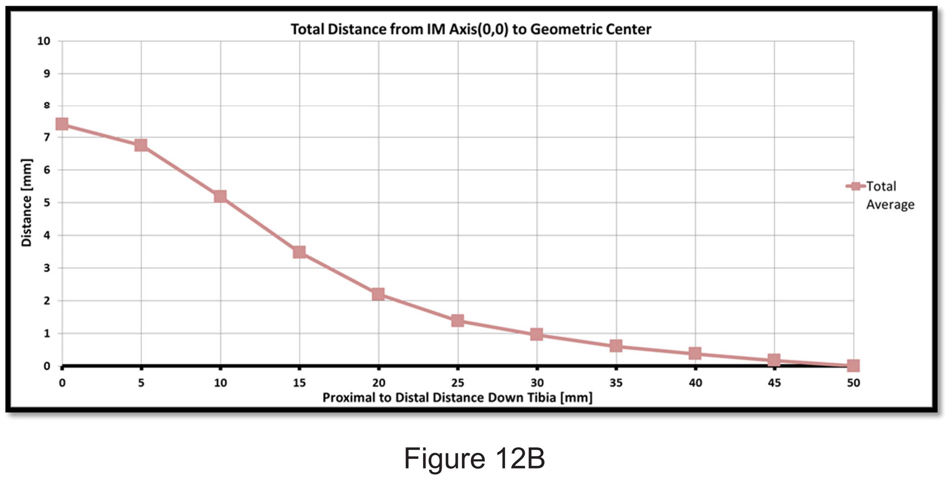

The total distance between the intramedullary points 710a to 710l and the geometric center points 720a to 720l, respectively, moving proximal to distal down the tibia was determined for all groups (see Figure 12A), as well as the total average (see Figure 12B)

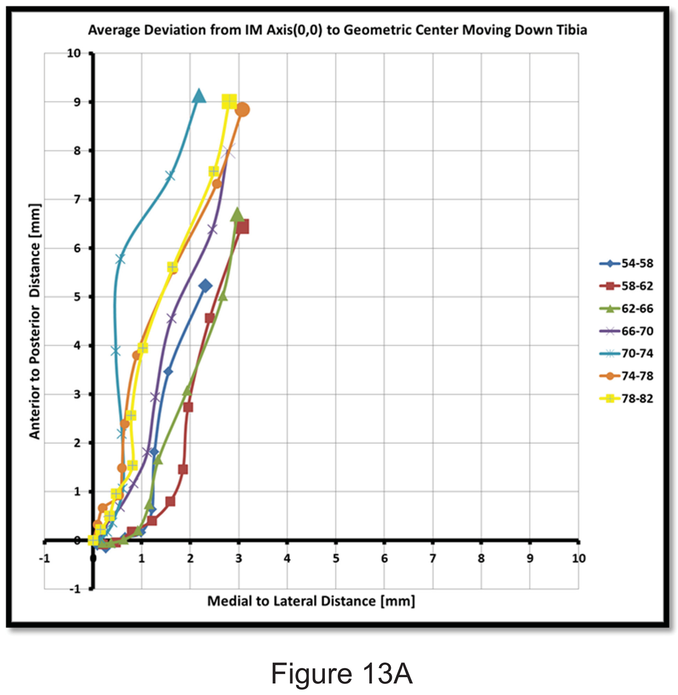

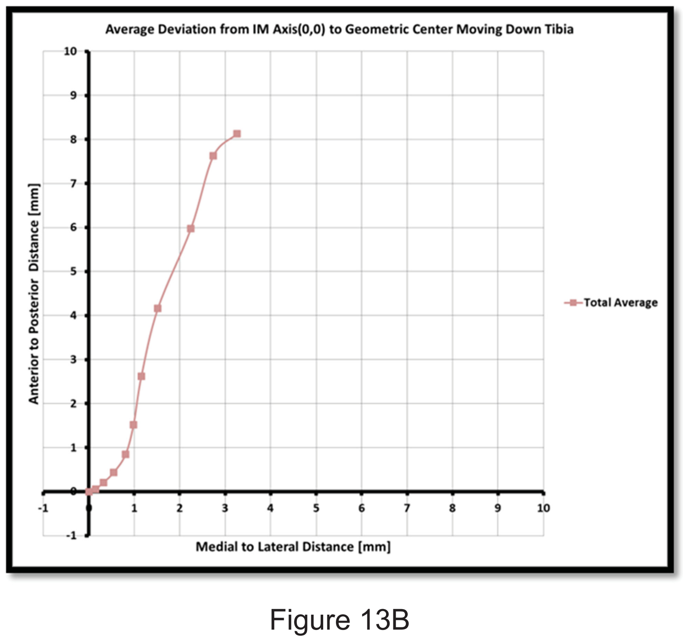

Graph demonstrating the average deviation from the intramedullary axis 530 to the geometric center points 720a to 720l of the tibia for all groups (see Figure 13A) and the total average (see Figure 13B).

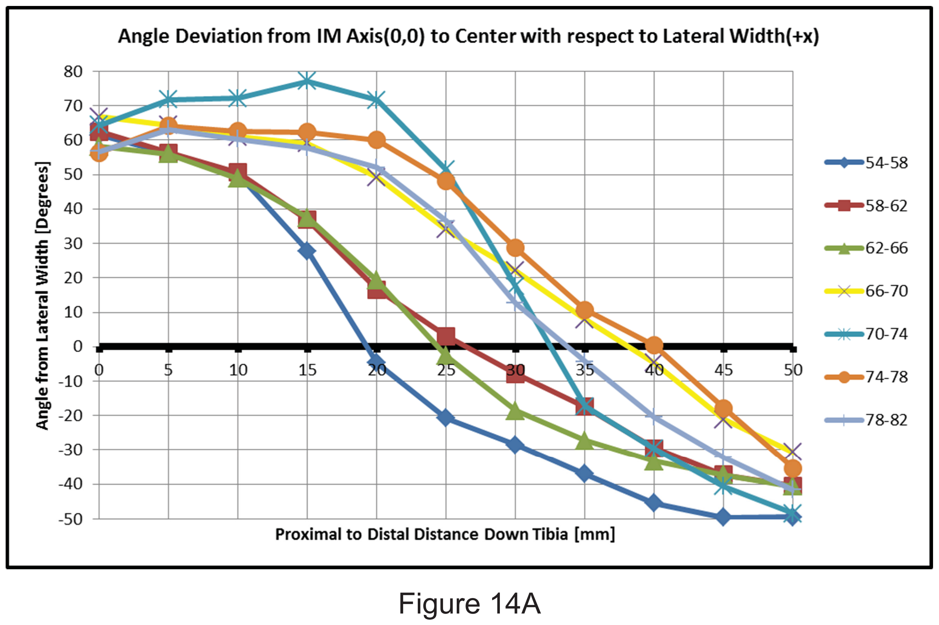

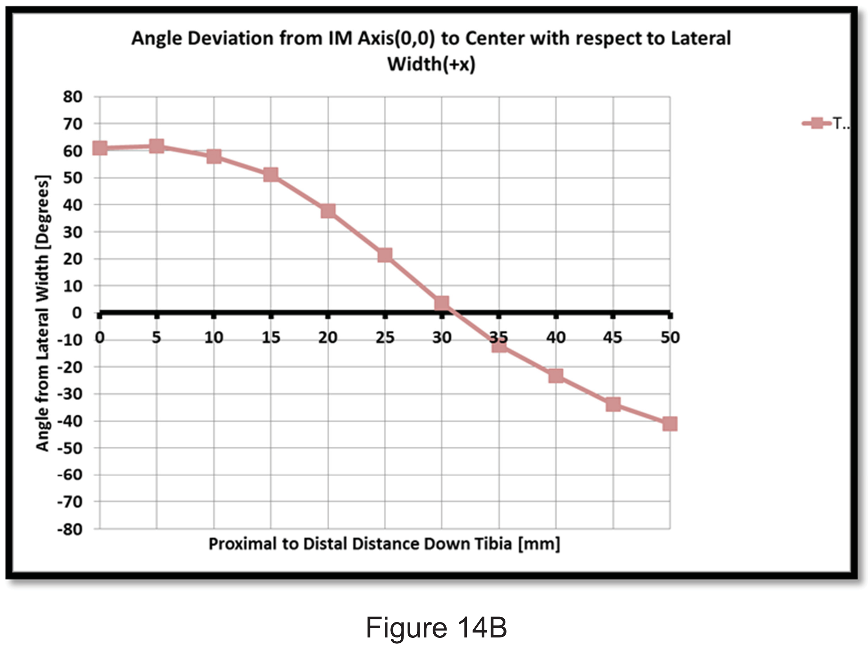

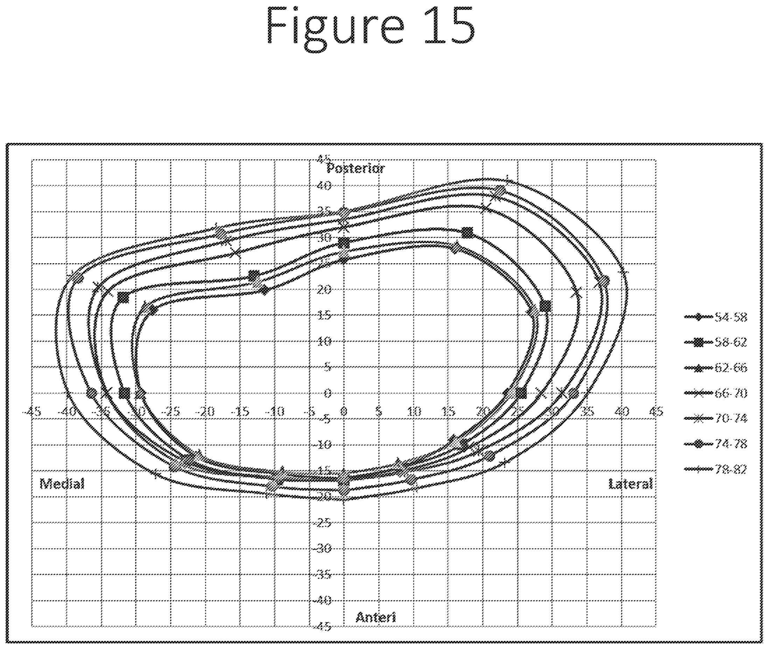

The angle deviation is a measurement of the angle between the intramedullary points 710a to 710l and the geometric center points 720a to 720l, respectively, and the x-axis for all groups (see Figure 14A) as well as the total average (see Figure 14B).
- Radial measurements of each of the groups outlines the contour of the tibia on the scans. See Figure 15.

### B. Distal Femoral Anatomy

### 1. Defining the Intramedullary Axis (IM Axis)

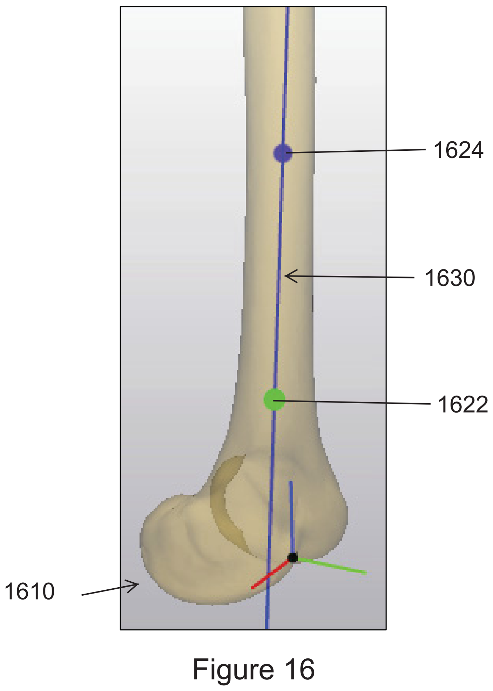

Looking at Figure 16, on the CT image of a femur 1610, a first plane and a second plane were created perpendicular to the femur at 75 mm. above the medial side of the femur 1610 and at 175 mm. above the medial side of the femur 1610. An inertial axis line was created, and a first intersection point 1622 and a second intersection point 1624 of the inertial axis line with the 75 mm. and 175 mm. surfaces respectively were drawn. An intramedullary axis 1630 was created by connecting these two points 1622, 1624. The intramedullary axis 1630 represents the center of the intramedullary canal in the distal femur.

### 2. Total Width Measurement

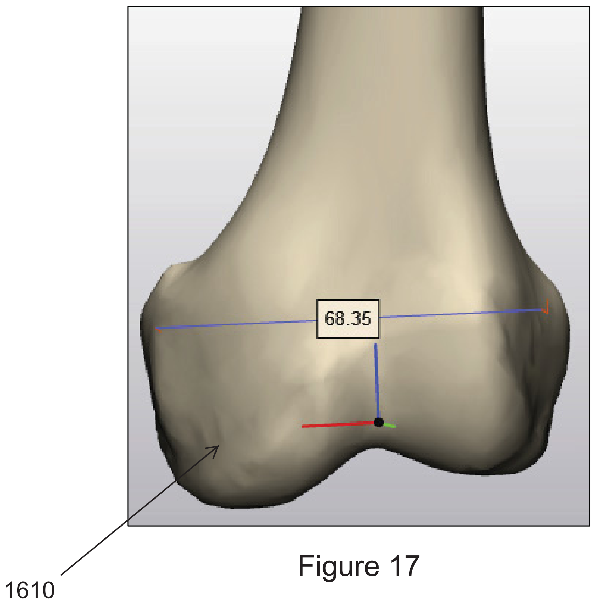

Referring to Figure 17, the total width (e.g., 68.35 mm. in Figure 17) was determined to be the distance between the medial and lateral epicondyles on the cancellous bone.

### 3. Deviation from Intramedullary (IM) Axis Measurement

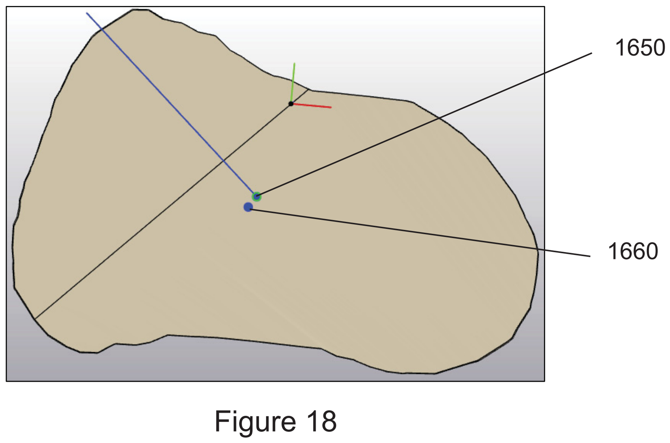

Referring to Figure 18, the distance between the intramedullary axis point 1650 (point of intersection between a slice and the IM axis) and the geometric center point 1660 was measured on a X-Y plane to quantify to the offset a knee sleeve would require.

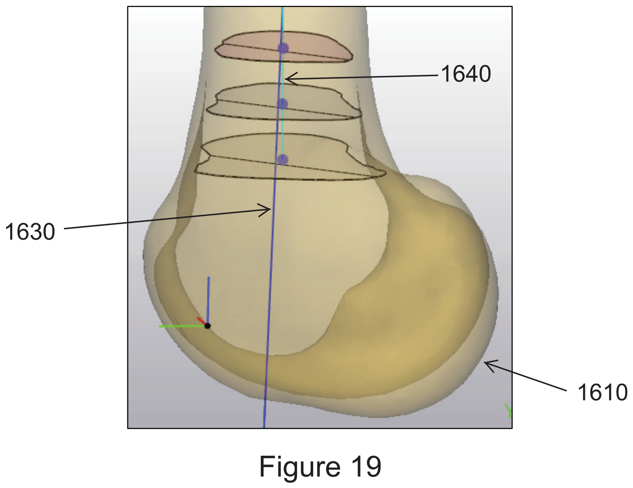

Figure 19 shows a visualization of the IM Axis 1630 and Geometric Centerline 1640 .

### 4. Radial Measurements

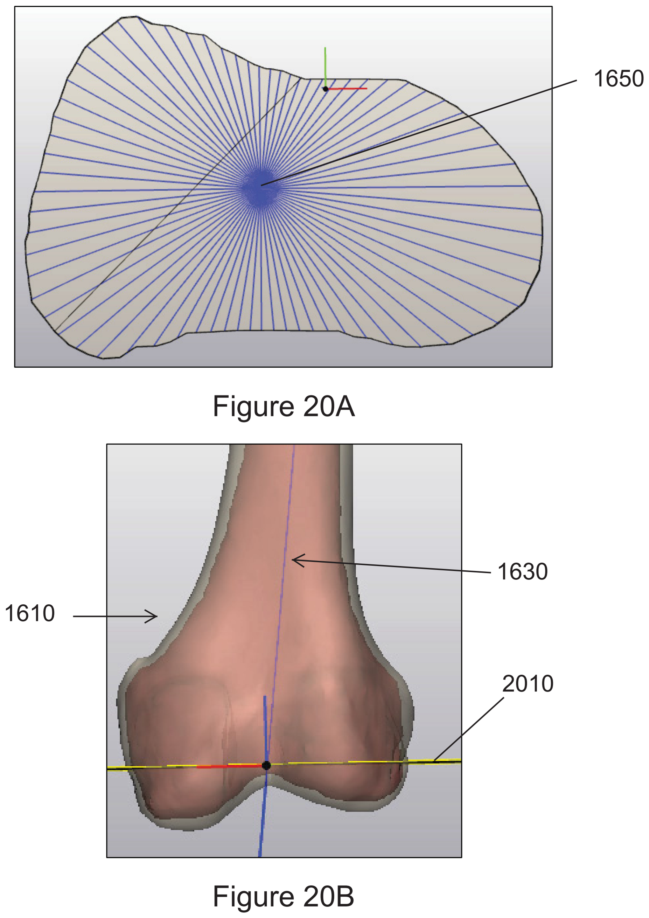

Looking at Figure 20A, on each slice a distance extending from the IM axis point 1650 to the edge of the cancellous bone was recorded every 5 degrees in order to find the average shape of each group.

### 5. Creating the Femoral Cut Plane

Referring to Figure 20B, the femoral cut plane 2010 was selected as the reference plane for all measurements. This was placed 10 mm. above the lowest point on the medial side of the femur 1610. The plane 2010 was tilted down anterior and medial 5 degrees with respect to the IM axis 1630.

### 6. Distal Femur Results

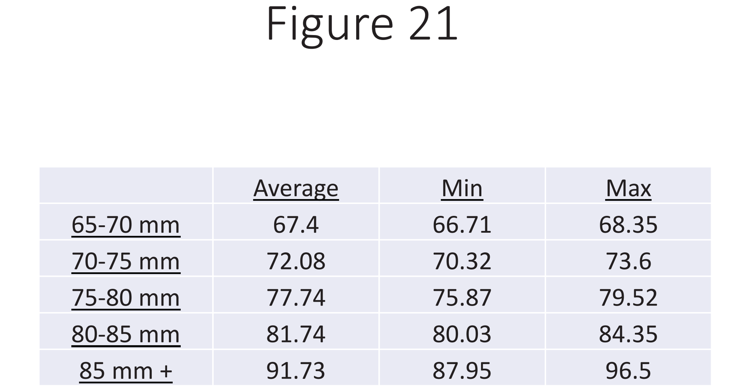

The medial-lateral width of the reference femur resection plane 2010 of the distal femur was placed into groups every 5 millimeters. The femurs were grouped by width: 65-70 mm., 71-75 mm., 75-80 mm., 80-85 mm., and 85+ mm. See Figure 21.

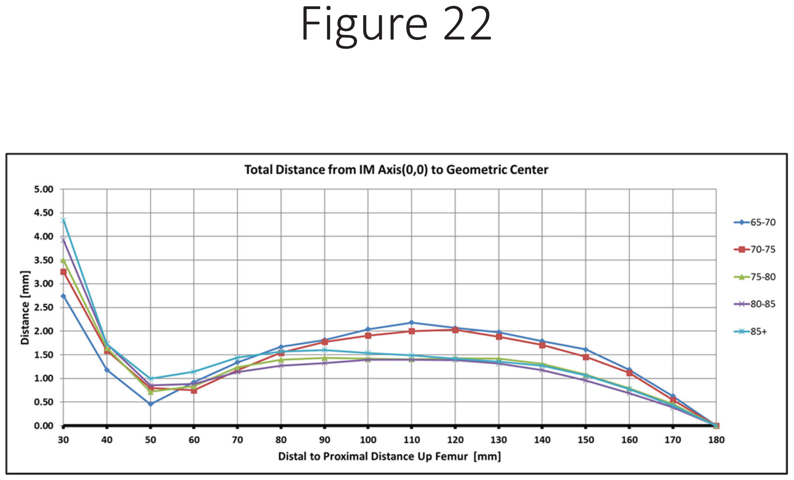

The total distance between IM axis point and the geometric center point moving distal to proximal up the femur was determined for all groups. See Figure 22.

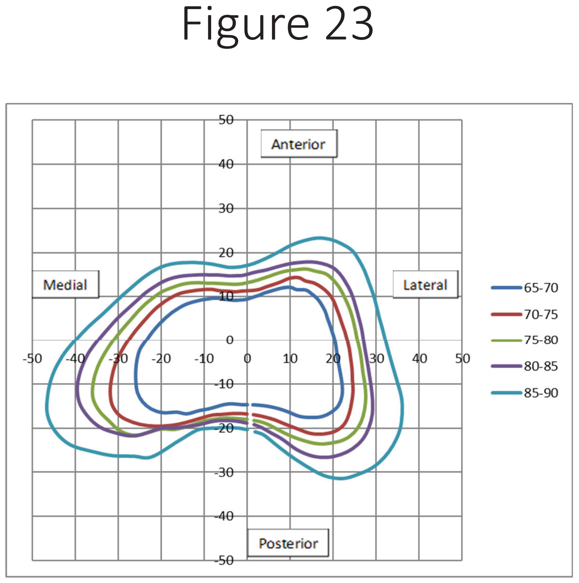

Radial measurements of each of the groups outlines the contour of the femur. See Figure 23.

### C. Results

The total deviation from the intramedullary axis point to the geometric center point on the scans moving down the tibia (proximal to distal) was substantial and confirms why surgeons have had challenges with the use of symmetric sleeves and straight stems in revision knee arthroplasty on the tibia. Additionally, there was a clear trend that the larger the tibia, the larger the total distance between these points. This provides insight on the specific amount of offset that should be designed into a tibia prosthesis based on the specific size of the proximal tibia width. See Figures 12A and 12B.

It can be seen that the deviation from the IM axis to the geometric center points as one moves distally in the tibia is an anteriorly and medial direction. See Figures 13A and 13B. The methodology demonstrates that the deviation occurs in multiple planes and that the cancellous curve does not follow a linear trend line.

The angle deviation is a measurement of the angle between the intramedullary axis point, the geometric center point, and the x-axis. See Figures 14A and 14B. The shift in location of the geometric center point and the intramedullary axis point demonstrate the specific angular change in this relationship as one moves distally in the tibia.

Radial measurements quantify the differences between the medial and lateral aspects of the proximal tibia. Figure 15 is an example of radial measurements made every 30 degrees at the most proximal tibial slice. This provides critical information for making appropriately sized and shaped sleeves.

For the femur, the total deviation from the intramedullary axis point to the geometric center point up the femur (distal to proximal) was also substantial and confirms why surgeons have had challenges with the use of symmetric sleeves and straight stems in revision knee arthroplasty on the femur. The maximum total distance is approximately 4.5 millimeters. This is at the most distal end of the femur which indicates that the greatest variation occurs at the distal end of the femur.

This is important to note because it indicates that the cancellous bone is not very linear. The distance reaches a local minimum at around 50 mm. from the cut plane then steadily increasing then decreasing to zero. The methodology helps determine the anatomic offset of the stem in relation to the distal femur. Additionally, the radial measurements demonstrated more deviation in the medial-lateral direction compared to anterior-posterior which is reflective of the sleeves that were designed.

### D. Implications for Component Design

The methodology confirms that the proximal tibial and distal femur regions are not symmetric in nature and clearly explains the challenges with forcing a circular or symmetric device in these regions. Additionally, the methodology demonstrates the clear difference between the architecture of the proximal medial versus the proximal lateral tibia as well as the distal medial versus distal lateral femur. In order to minimize medial overhang of the tibial tray, many surgeons are forced to significantly downsize the tibial component which impacts the kinematics of the joint. Moreover, systems mandate that the size of the femoral component needs to be similar to the size of the tibial component. This forced undersizing of the tibial component, then forces the surgeon to use a non-optimal size of the femoral component. This methodology drives the design of anatomically shaped sleeves to address bone deficiencies in these regions.

The methodology helps to clearly define the offset between the center of the tibia and femur at the joint line versus the center of the distal canal where the stem is placed. Currently available systems do not take this properly into account. The methodology allows the design of implants that accommodate this offset in magnitude and angular direction.

The methodology clearly demonstrates the need for different sleeves on the medial and lateral side. Moreover, the radial measurements provide key insight into exactly how the sleeves should grow in the medial-lateral vs. anterior-posterior direction as one increases in size.

### E. Application of the Methodology and Creation of

### An Anatomic System for Revision Knee Arthroplasty

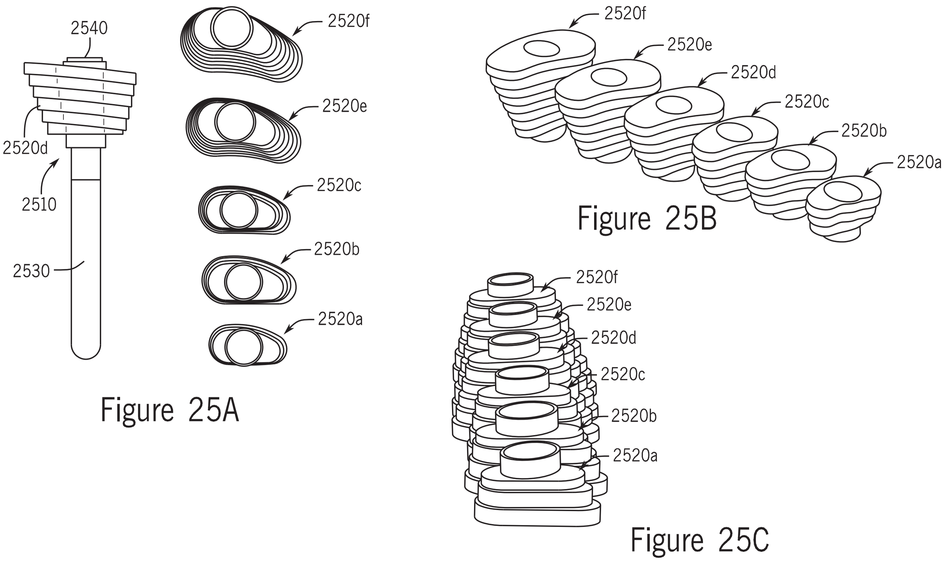

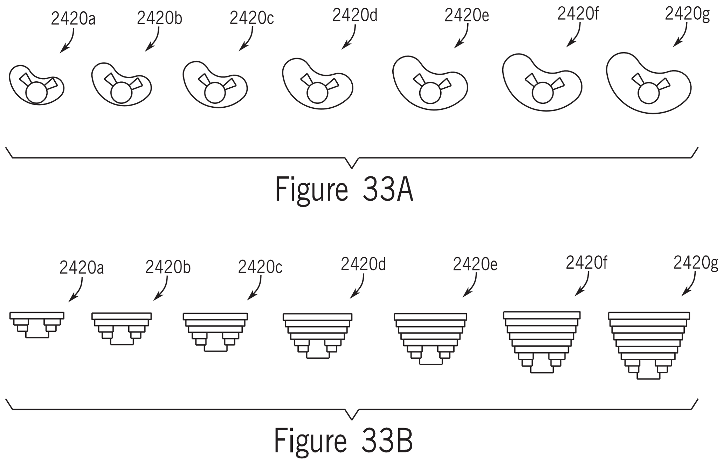

The methodology resulted in the development of a complete suite of anatomically shaped sleeves or cones for tibial and femoral reconstruction for revision knee arthroplasty. Moreover, the specific orientation of the stems in relation to the sleeve or cone were driven by science and a true anatomic basis. Seven tibial sleeves (2420a to 2420g (see Figure 33A) and six femoral sleeves 2520a to 2520f (see Figure 25B) were created based on the distribution of anatomic measurements that optimized contact with underlying bone. Similar size distributions were created for cones. A complete set with broaches, sleeves, adapters, cones, and stems was created. The size, shape, and location of the femoral sleeves and the tibial sleeves facilitate reconstruction resulting in maximum bone preservation and optimizing contact with host bone. Similarly, the size, shape, and location of the femoral cones and the tibial cones facilitate reconstruction resulting in maximum bone preservation and optimizing contact with host bone. The offset of the stems in relation to the sleeves and cones was driven by the methodology allowing for optimized fit and minimizing the risk of iatrogenic fracture.

### Non-Limiting Example Sleeves

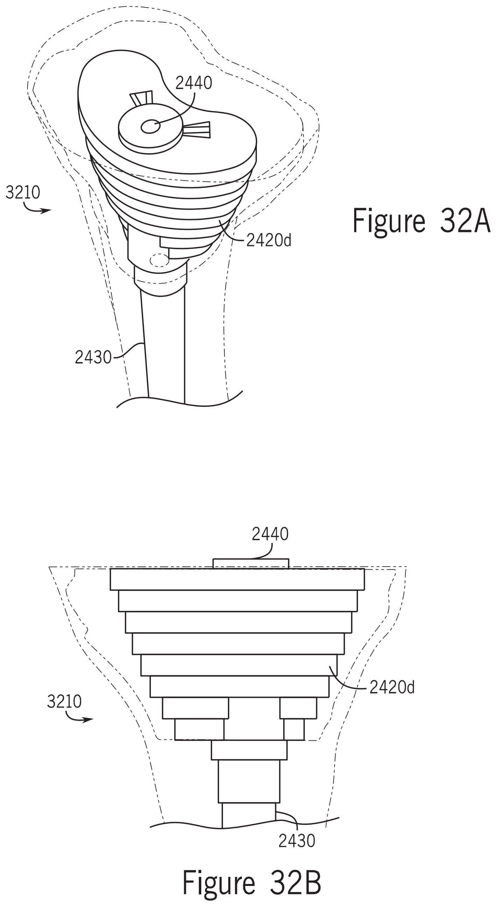

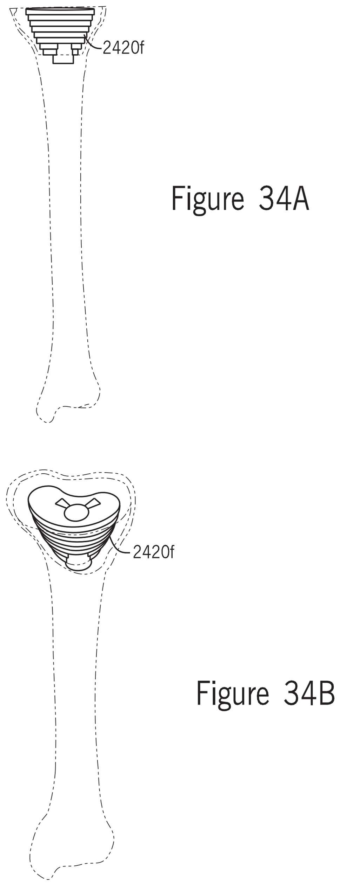

Looking at Figures 32A and 32B, there is shown a tibial component 3210 including a sleeve 2420d, a stem 2430, and an adapter 2440. One can see how this offset of the stem 2430 facilitates the stem 2430 to be placed down the center of the tibial canal. Additionally, the sleeves grow incrementally larger medially compared to laterally which is consistent with the underlying anatomy. One can see how the asymmetrical shape of the tibial sleeve maximizes contact with the underlying bone. See Figures 33A, 33B, 34A and 34B.

In Figures 32A and 32B, the opening in the sleeve 2420d for the stem 2430 is offset in an anterior and medial direction relative to the geometric center point of the cancellous bone at the proximal aspect of the tibia.

In Figure 33A, the sleeves 2420a to 2420g grow incrementally greater medially compared to laterally which is consistent with the underlying anatomy.

In Figure 33B, the tibial sleeves 2420a to 2420g increase in size by adding an incremental step which makes moving up and down sizes intuitive and the instrumentation streamlined.

In Figures 34A and 34B, the tibial sleeve 2420d design matches the underlying anatomy preserving bone and optimizing implant-bone surface area contact for long term ingrowth.

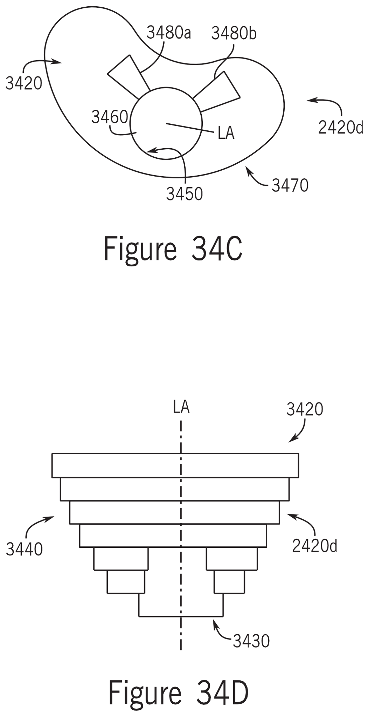

Figures 34C and 34D show an embodiment of a tibial sleeve 2420d according to the invention. The sleeve 2420d has a first end surface 3420, a second end surface 3430, an exterior surface 3440 extending from the first end surface 3420 to the second end surface 3430, and an inner surface 3450 defining a passageway 3460 extending from the first end surface 3420 to the second end surface 3430, wherein the exterior surface 3440 of the sleeve 2420d is configured to be received in a cavity of the tibia such that the first end surface 3420 is flush with or beneath a surface of the resected tibia. The first end surface 3420 includes locating slots 3480a and 3480b that are dimensioned to receive stabilization arms of a stem. The first end surface 3420 is within an axial plane defined by the first end surface 3420 and an outermost edge 3470 (in this embodiment, a perimeter) of the first end surface 3420. A longitudinal axis LA of the passageway 3460 of the sleeve 2420d is offset with respect to a geometric center point of the axial plane. The longitudinal axis LA of the passageway 3460 of the sleeve 2420d can correspond to an intramedullary axis as determined above with reference to Figure 5.

The exterior surface of the tibial sleeve 2420d can have a variety of surfaces including smooth, roughened, grit blasted, and have porous ingrowth material for bone ingrowth. For example, open cell tantalum structures have been developed for potential application in reconstructive orthopedics and other surgical disciplines. The material has high and interconnected porosity with a very regular pore shape and size. It can be made into complex shapes as a surface coating. "Trabecular metal" has been shown to permit physiologic bone in growth and healing. Also, the exterior surface of the tibial sleeve 2420d can be modified by sand or bead blasting the surface, or otherwise roughening the surfaces in some way. The surface may also be modified by shaping the surface, such as by using blades, pointed structures, machining lines or a geometric feature on the exterior surface.

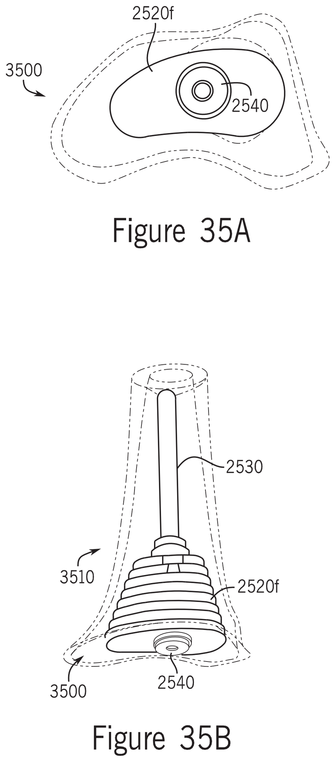

The tibial sleeve 2420d can be used in a prosthetic system comprising a tibial implant having a body and an attached stem extending away from the body, wherein the stem 2430 is positioned within the passageway 3460 of the sleeve 2420d as in Figure 32B. The tibial sleeve 2420d can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component. The femoral sleeve can be used in a prosthetic system comprising a femoral component having medial and lateral condyles with curved articulating surfaces as in Figure 1. The femoral sleeve 2520d can be used in a prosthetic system comprising a femoral implant having a body and an attached stem 2530 extending away from the body, wherein the stem 2530 is positioned within the passageway 3660 of a femoral sleeve 2520d as in Figure 35B.

In currently commercialized revision knee systems, this stem offset has not been properly accounted for in the design and causes two significant clinical problems:
(1) If the stem is placed down the center of the canal, the current symmetric sleeves on the market will be unable to optimally contact the remaining bone. This results in increase stress on the construct increasing the chances of failure.
(2) If the surgeon places the symmetric sleeve asymmetrically in the proximal tibial region to engage the natural bony architecture, the surgeon is forced to place the stem eccentrically in the canal leading to malalignment of the implant.

Both of these commonly encountered problems are obviated with the design of the present invention representing a significant step forward by aligning the stem centrally within the canal and then having the sleeves and cones grow where the bone is located.

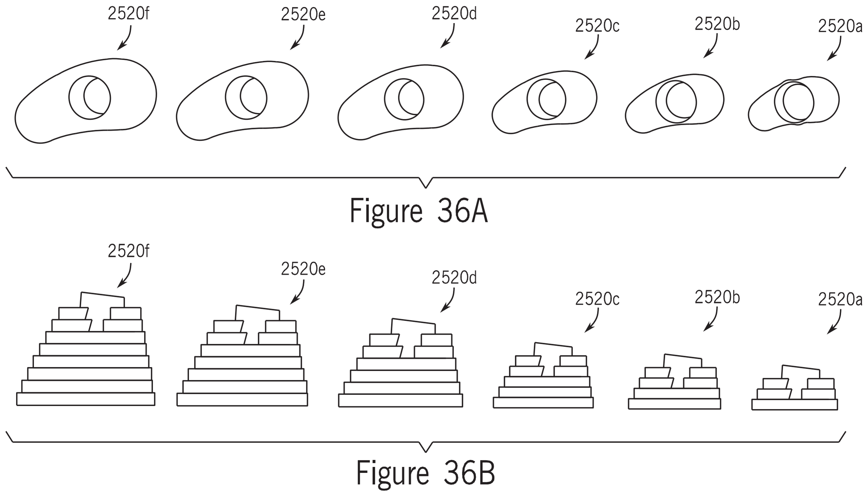

The present invention provides a system that maximizes bone preservation, with sleeves and cones that logically increase in sizes with incremental steps. One can see a design philosophy on the femur 3500 that is similar to the tibia with anatomically shaped sleeves that optimize contact with the underlying bone. In Figures 35A and 35B, there is shown a femoral component 3510 including a sleeve 2520d, a stem 2530, and an adapter 2540. The femoral sleeve 2520d design matches the underlying anatomy preserving bone and optimizing the implant. Figure 36A shows top views of femoral sleeves 2520a to 2520f wherein the opening in the femoral sleeve for the stem has a 6 degree angle corresponding to the canal of the femur. Figure 36B shows anterior views of the femoral sleeves 2520a to 2520f wherein the femoral sleeves 2520a to 2520f increase in size by adding an incremental step which makes moving up and down sizes intuitive and the instrumentation streamlined. The femoral sleeves 2520a to 2520f have a 6 degree angle that matches the femoral canal and improves sizing options.

The exterior surface of any of the femoral sleeves 2520a to 2520f can have a variety of surfaces including smooth, roughened, grit blasted, and have porous ingrowth material for bone ingrowth. For example, open cell tantalum structures have been developed for potential application in reconstructive orthopedics and other surgical disciplines. The material has high and interconnected porosity with a very regular pore shape and size. It can be made into complex shapes as a surface coating. "Trabecular metal" has been shown to permit physiologic bone in growth and healing. Also, the exterior surface of the femoral sleeves 2520a to 2520f can be modified by sand or bead blasting the surface, or otherwise roughening the surfaces in some way. The surface may also be modified by shaping the surface, such as by using blades, pointed structures, machining lines or a geometric feature on the exterior surface.

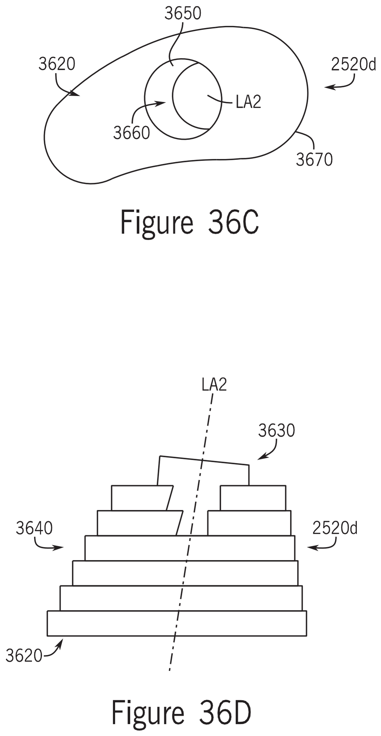

Figures 36C and 36D show an embodiment of a femoral sleeve 2520d according to the invention. The sleeve 2520d has a first end surface 3620, a second end surface 3630, an exterior surface 3640 extending from the first end surface 3620 to the second end surface 3630, and an inner surface 3650 defining a passageway 3660 extending from the first end surface 3620 to the second end surface 3630, wherein the exterior surface 3640 of the sleeve 2520d is configured to be received in a cavity of the femur such that the first end surface 3620 is flush with or beneath a surface of the resected femur. The first end surface 3620 is within an axial plane defined by the first end surface 3620 and an outermost edge 3670 (in this embodiment, a perimeter) of the first end surface 3620. A longitudinal axis LA2 of the passageway 3660 of the sleeve 2520d is offset with respect to a geometric center point of the axial plane. The longitudinal axis LA2 of the passageway 3660 of the sleeve 2520d can correspond to an intramedullary axis as determined above with reference to Figure 16.

In the femoral sleeve 2520d, the longitudinal axis LA2 of the passageway 3660 of the sleeve 2520d can be angled at an oblique angle with respect to a normal line to the axial plane defined by the first end surface 3620 and the outermost edge 3670 of the first end surface 3620. The oblique angle can be greater than 0 degrees and less than 10 degrees. The oblique angle can be greater than 3 degrees and less than 9 degrees. The oblique angle can be 5 or 6 or 7 degrees.

The femoral sleeve 2520d can be used in a prosthetic system comprising a femoral component having medial and lateral condyles with curved articulating surfaces as in Figure 1. The femoral sleeve 2520d can be used in a prosthetic system comprising a femoral implant having a body and an attached stem 2530 extending away from the body, wherein the stem 2530 is positioned within the passageway 3660 of the sleeve 2520d as in Figure 35B. The femoral sleeve 2520d can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

A breadth of sizes greatly broadens the potential for use of sleeves in revision knee arthroplasty. Current sleeves have limited sizes with large changes between sizes forcing implants that are too small and do not maximize contact or necessitate additional bone removal to make them fit. The availability of smaller sleeve sizes in the invention also broadens the market for use of sleeves which is a premium product with premium pricing which is attractive to manufacturers.

Additionally, the system and implants create a streamlined surgical flow. For the tibia, the distal sizing is confirmed with cylindrical reamers and then broaches for the sleeves are utilized to ensure maximum support and minimize bone removal. The same is true for the femur after engaging with proximal femoral diaphyseal bone. The system of the invention allows any size sleeve to be used with any size stem. This greatly facilitates broadening the use of sleeves in revision cases and expands the market opportunity. For example, a small sleeve can be used in any size patient providing a better option compared to bone graft, cement, or removing additional bone to make current non-anatomically shaped and sized sleeves fit.

The sleeves may be formed from a metal alloy such as titanium alloys (e.g., titanium-6-aluminum-4-vanadium), cobalt-chromium alloys, stainless steel alloys and tantalum alloys; nonresorbable ceramics such as aluminum oxide and zirconia; nonresorbable polymeric materials such as polyethylene; or composite materials such as carbon fiber-reinforced polymers (e.g., polysulfone). Preferably, the sleeve is formed from a tantalum based porous material, or it may be made from another metal that is coated with a tantalum-based porous metal or other porous coating.

### Non-Limiting Example Cones

The outer shape and diameter of cones may be similar to those of sleeves. The inner diameter of the cone may be wider than the inner diameter of a sleeve. In some configurations, both the proximal and distal openings in the cone are wider than that of a sleeve. This allows a wider range of positions and angles to place the stem through the cone compared to the sleeve.

The cones can be created by using the same methodology as the sleeves and then removing material from the inside to create a large inner diameter. The anatomic shape and offset for the stem may be similar in both cases and based on the methodology described.

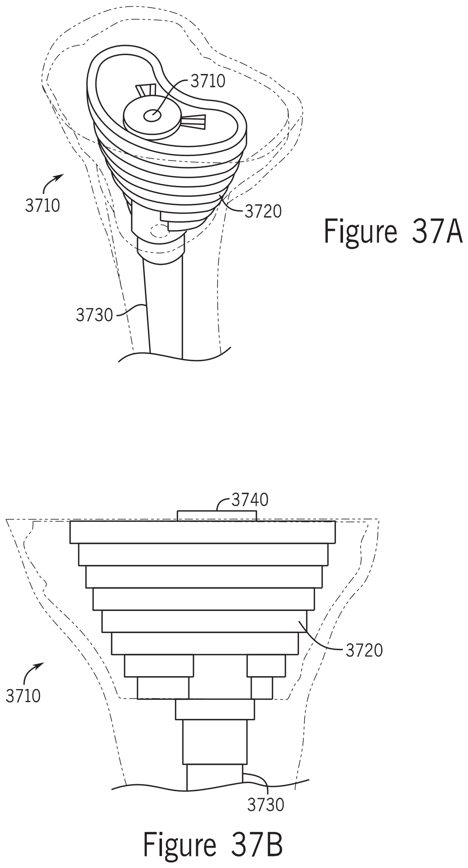

Looking at Figures 37A and 37B, there is shown a tibial component 3710 including a cone 3720, a stem 3730, and an adapter 3740. One can see how this offset of the stem 3730 facilitates the stem 3730 to be placed down the center of the tibial canal. Additionally, the cones grow incrementally larger medially compared to laterally which is consistent with the underlying anatomy. One can see how the asymmetrical shape of the tibial cones maximizes contact with the underlying bone. See Figures 33A, 33B, 34A and 34B.

In Figures 37A and 37B, the opening in the cone 3720 for the stem 2730 is offset in an anterior and medial direction relative to the geometric center point of the cancellous bone at the proximal aspect of the tibia.

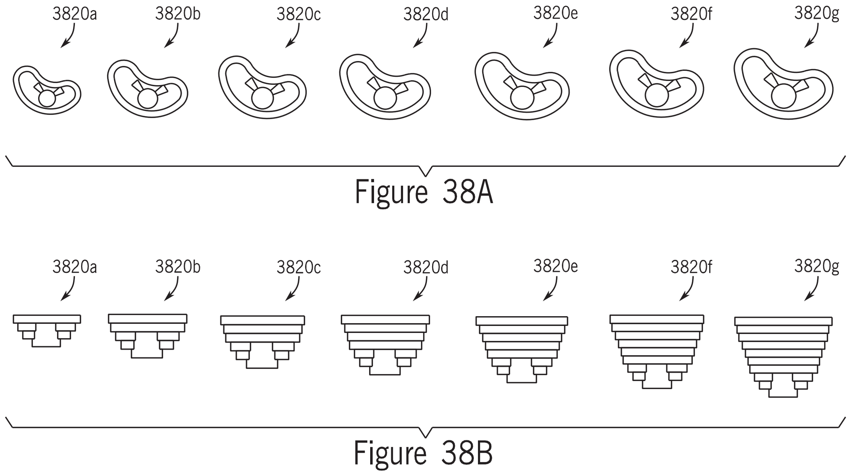

In Figure 38A, the cones 3820a to 3820g grow incrementally greater medially compared to laterally which is consistent with the underlying anatomy.

In Figure 38B, the tibial cones 3820a to 3820g increase in size by adding an incremental step which makes moving up and down sizes intuitive and the instrumentation streamlined.

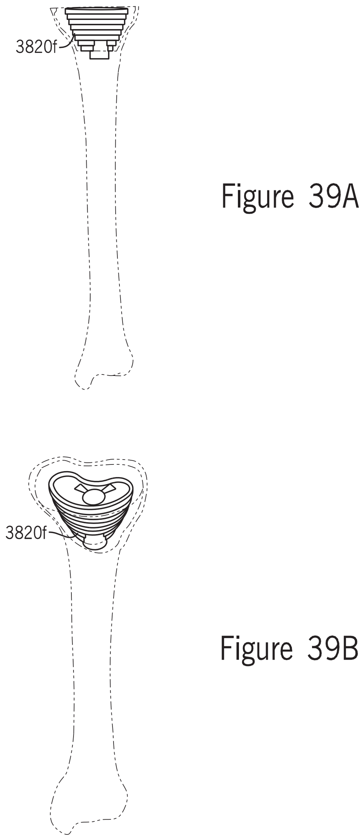

In Figures 39A and 39B, the tibial cone 3820f design matches the underlying anatomy preserving bone and optimizing implant-bone surface area contact for long term ingrowth.

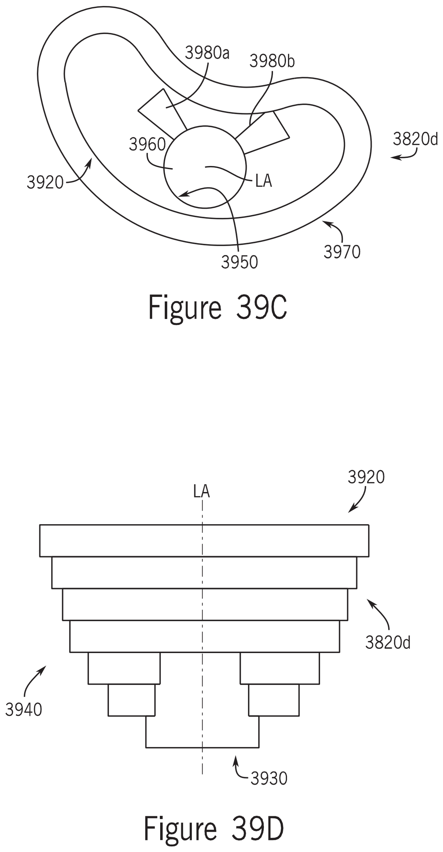

Figures 39C and 39D show an embodiment of a tibial cone 3820d according to the invention. The cone 3820d has a first end surface 3920, a second end surface 3930, a stepped exterior surface 3940 extending from the first end surface 3920 to the second end surface 3930, and an inner surface 3950 defining a passageway 3960 extending from the first end surface 3920 to the second end surface 3930, wherein the exterior surface 3940 of the cone 3820d is configured to be received in a cavity of the tibia such that the first end surface 3920 is flush with or beneath a surface of the resected tibia. A stem for use with the cone may include stabilization arms 3980a and 3980b. The first end surface 3920 is within an axial plane defined by the first end surface 3920 and an outermost edge 3970 (in this embodiment, a perimeter) of the first end surface 3920. A longitudinal axis LA of the passageway 3960 of the cone 3820d is offset with respect to a geometric center point of the axial plane. The longitudinal axis LA of the passageway 3960 of the cone 3820d can correspond to an intramedullary axis as determined above with reference to Figure 5.

The tibial cone 3820d can be used in a prosthetic system comprising a tibial implant having a body and a stem extending away from the body, wherein the stem is positioned within the passageway 3960 of the cone 3820d as in Figure 37B. The tibial cone 3820d can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component. The cone can be used in a prosthetic system comprising a femoral component having medial and lateral condyles with curved articulating surfaces as in Figure 1.

The exterior surface of any of the tibial cones 3820a to 3820g can have a variety of surfaces including smooth, roughened, grit blasted, and have porous ingrowth material for bone ingrowth. For example, open cell tantalum structures have been developed for potential application in reconstructive orthopedics and other surgical disciplines. The material has high and interconnected porosity with a very regular pore shape and size. It can be made into complex shapes as a surface coating. "Trabecular metal" has been shown to permit physiologic bone in growth and healing. Also, the exterior surface of the tibial cones 3820a to 3820g can be modified by sand or bead blasting the surface, or otherwise roughening the surfaces in some way. The surface may also be modified by shaping the surface, such as by using blades, pointed structures, machining lines or a geometric feature on the exterior surface.

Both of the commonly encountered problems for stem offset failures noted above with sleeves may also be obviated with the design of the present invention representing a significant step forward by aligning the stem centrally within the canal and then having the cones grow where the bone is located.

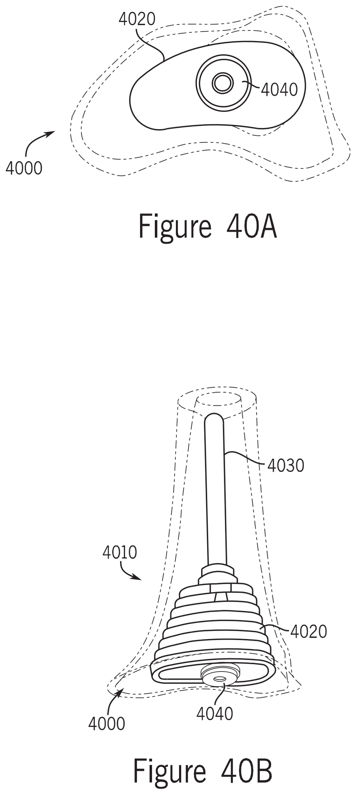

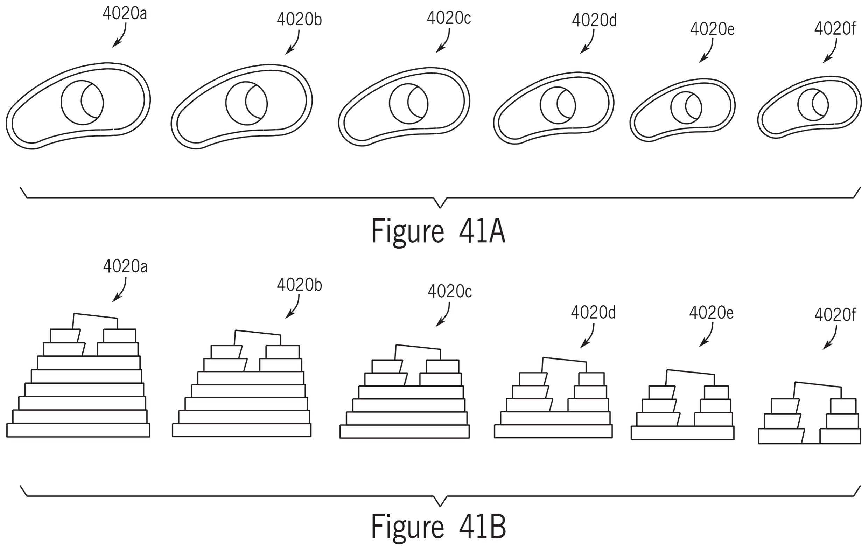

The present invention provides a system that maximizes bone preservation, with sleeves and cones that logically increase in sizes with incremental steps. One can see a design philosophy on the femur 4000 that is similar to the tibia with anatomically shaped cones that optimize contact with the underlying bone. In Figures 40A and 40B, there is shown a femoral component 4010 including a cone 4020, a stem 4030, and an adapter 4040. The femoral cone 4020 design matches the underlying anatomy preserving bone and optimizing the implant. Figure 41A shows top views of femoral cones 4020a to 4020f wherein the opening in the femoral cone for the stem has a 6 degree angle corresponding to the canal of the femur. Figure 41B shows anterior views of the femoral cones 4020a to 4020f wherein the femoral cones 4020a to 4020f increase in size by adding an incremental step which makes moving up and down sizes intuitive and the instrumentation streamlined. The femoral cones 4020a to 4020f have a 6 degree angle that matches the femoral canal and improves sizing options.

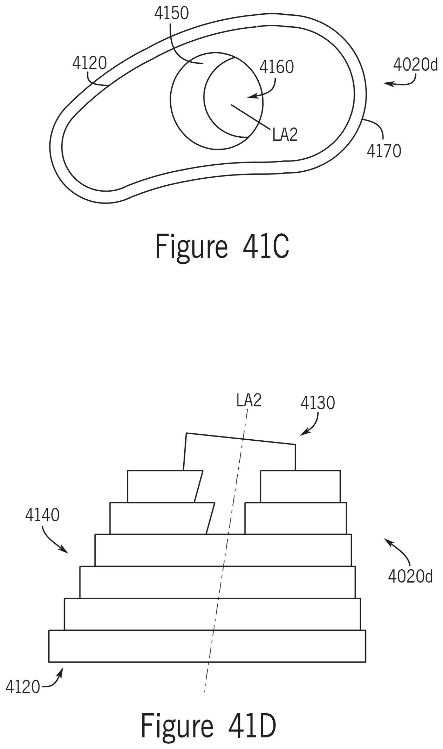

Figures 41C and 41D show an embodiment of a femoral cone 4020d according to the invention. The cone 4020d has a first end surface 4120, a second end surface 4130, a stepped exterior surface 4140 extending from the first end surface 4120 to the second end surface 4130, and an inner surface 4150 defining a passageway 4160 extending from the first end surface 4120 to the second end surface 4130, wherein the exterior surface 4140 of the cone 4020d is configured to be received in a cavity of the femur such that the first end surface 4120 is flush with or beneath a surface of the resected femur. The first end surface 4120 is within an axial plane defined by the first end surface 4120 and an outermost edge 4170 of the first end surface 4120. A longitudinal axis LA2 of the passageway 4160 of the cone 4020d is offset with respect to a geometric center point of the axial plane. The longitudinal axis LA2 of the passageway 4160 of the cone 4020d can correspond to an intramedullary axis as determined above with reference to Figure 16.

In the femoral cone 4020d, the longitudinal axis LA2 of the passageway 4160 of the cone 4020d can be angled at an oblique angle with respect to a normal line to the axial plane defined by the first end surface 4120 and the outermost edge 4170 (in this embodiment, a perimeter) of the first end surface 4120. The oblique angle can be greater than 0 degrees and less than 10 degrees. The oblique angle can be greater than 3 degrees and less than 9 degrees. The oblique angle can be 5 or 6 or 7 degrees.

The exterior surface of any of the femoral cones 4020a to 4020f can have a variety of surfaces including smooth, roughened, grit blasted, and have porous ingrowth material for bone ingrowth. For example, open cell tantalum structures have been developed for potential application in reconstructive orthopedics and other surgical disciplines. The material has high and interconnected porosity with a very regular pore shape and size. It can be made into complex shapes as a surface coating. "Trabecular metal" has been shown to permit physiologic bone in growth and healing. Also, the exterior surface of the femoral cones 4020a to 4020f can be modified by sand or bead blasting the surface, or otherwise roughening the surfaces in some way. The surface may also be modified by shaping the surface, such as by using blades, pointed structures, machining lines or a geometric feature on the exterior surface.

The femoral cone 4020d can be used in a prosthetic system comprising a femoral component having medial and lateral condyles with curved articulating surfaces as in Figure 1. The femoral cone 4020d can be used in a prosthetic system comprising a femoral implant having a body and a stem extending away from the body, wherein the stem is positioned within the passageway 4160 of the cone 4020d as in Figure 40B. The femoral cone 4020d can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

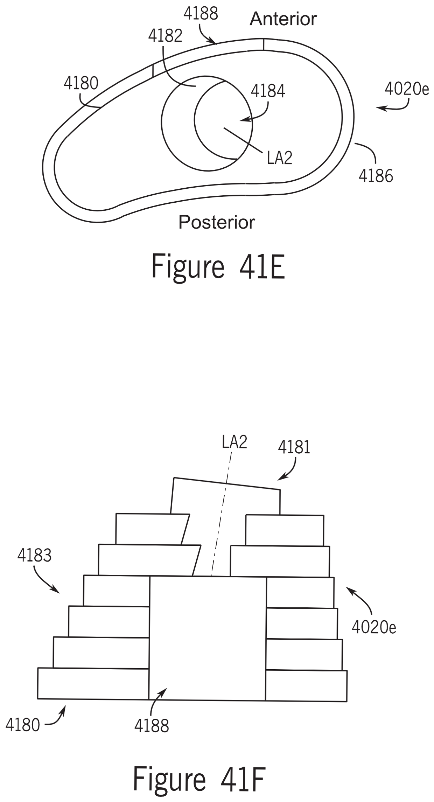

Figures 41E and 41F show another embodiment of a femoral cone 4020e according to the invention having a portion of the anterior wall removed to create a notch. The cone 4020e has a first end surface 4180, a second end surface 4181, a stepped exterior surface 4183 extending from the first end surface 4180 to the second end surface 4181, and an inner surface 4182 defining a passageway 4184 extending from the first end surface 4180 to the second end surface 4181, wherein the exterior surface 4183 of the cone 4020e is configured to be received in a cavity of the femur such that the first end surface 4180 is flush with or beneath a surface of the resected femur. A notch 4188 is in the anterior wall of the cone 4020e. Removing a portion of the anterior wall accommodates the bow of the femur which increases sagittal plane freedom when placing a femoral stem. The first end surface 4180 is within an axial plane defined by the first end surface 4180 and an outermost edge 4186 of the first end surface 4180. A longitudinal axis LA2 of the passageway 4184 of the cone 4020e is offset with respect to a geometric center point of the axial plane. The longitudinal axis LA2 of the passageway 4184 of the cone 4020e can correspond to an intramedullary axis as determined above with reference to Figure 16.

In the femoral cone 4020e, the longitudinal axis LA2 of the passageway 4184 of the cone 4020e can be angled at an oblique angle with respect to a normal line to the axial plane defined by the first end surface 4180 and the outermost edge 4186 of the first end surface 4180. The oblique angle can be greater than 0 degrees and less than 10 degrees. The oblique angle can be greater than 3 degrees and less than 9 degrees. The oblique angle can be 5 or 6 or 7 degrees.

The femoral cone 4020e can be used in a prosthetic system comprising a femoral component having medial and lateral condyles with curved articulating surfaces as in Figure 1. The femoral cone 4020e can be used in a prosthetic system comprising a femoral implant having a body and a stem extending away from the body, wherein the stem is positioned within the passageway 4184 of the cone 4020e as in Figure 40B. The femoral cone 4020e can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

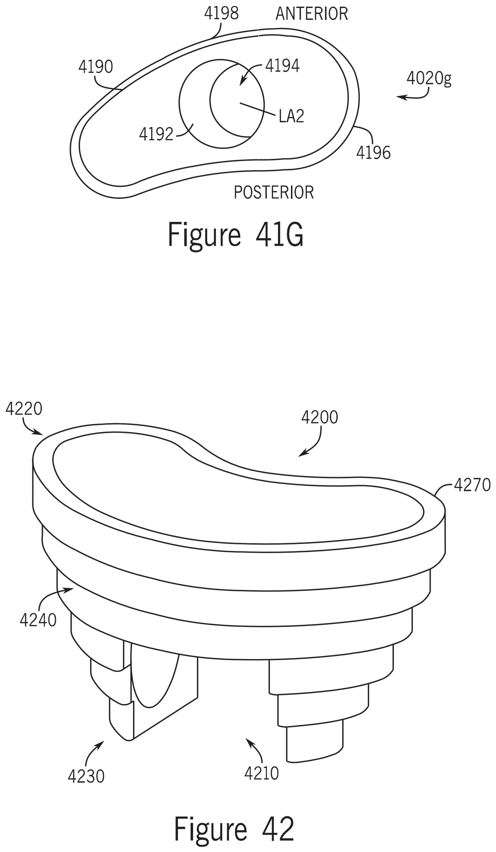

Figure 41G shows a top view of yet another embodiment of a femoral cone 4020g according to the invention wherein the anterior wall of the femoral cone is thinner compared to the lateral and medial walls. The cone 4020g has a first end surface 4190, a second end surface (similar to the second end surface 4181 in Figures 41E and 41F), an exterior surface (similar to exterior surface 4183 in Figures 41E and 41F) extending from the first end surface 4190 to the second end surface, and an inner surface 4192 defining a passageway 4194 extending from the first end surface 4190 to the second end surface, wherein the exterior surface of the cone 4020g is configured to be received in a cavity of the femur such that the first end surface 4190 is flush with or beneath a surface of the resected femur. A thinner wall section 4198 is in the anterior wall of the cone 4020g. A thinned portion of the anterior wall accommodates the bow of the femur which increases sagittal plane freedom when placing a femoral stem. The first end surface 4190 is within an axial plane defined by the first end surface 4190 and an outermost edge 4196 of the first end surface 4190. A longitudinal axis LA2 of the passageway 4194 of the cone 4020g is offset with respect to a geometric center point of the axial plane. The longitudinal axis LA2 of the passageway 4194 of the cone 4020g can correspond to an intramedullary axis as determined above with reference to Figure 16.

In the femoral cone 4020g, the longitudinal axis LA2 of the passageway 4194 of the cone 4020g can be angled at an oblique angle with respect to a normal line to the axial plane defined by the first end surface 4190 and the outermost edge 4196 of the first end surface 4190. The oblique angle can be greater than 0 degrees and less than 10 degrees. The oblique angle can be greater than 3 degrees and less than 9 degrees. The oblique angle can be 5 or 6 or 7 degrees.

The femoral cone 4020g can be used in a prosthetic system comprising a femoral component having medial and lateral condyles with curved articulating surfaces as in Figure 1. The femoral cone 4020g can be used in a prosthetic system comprising a femoral implant having a body and a stem extending away from the body, wherein the stem is positioned within the passageway 4194 of the cone 4020g as in Figure 40B. The femoral cone 4020g can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

In some sizes of the cone according to the invention, such as smaller sizes, a portion of the distal anterior and posterior wall may be removed to accommodate a large size stem as well as to allow for placing the stem in a range of positions and angulations. This can be seen in Figure 42, with removed wall portion forming a notch 4210 of the tibial cone 4200. Instead of a fixed position as in the case of a sleeve, the wider proximal and distal openings of the cone provide for the stem to be selectively oriented or angled with respect to the cone without the stem impinging upon the interior edge of a cone, and the notch 4210 may provide for a relief for the lower portion of a stem as the orientation or angle of the stem is changed with respect to the longitudinal axis of the cone such that the lower portion of the stem does not impinge or bind against the narrower portion of the cone. Looking at Figure 42, the tibial cone 4200 has a first end surface 4220, a second end surface 4230, a stepped exterior surface 4240 extending from the first end surface 4220 to the second end surface 4230, and an inner surface defining a passageway extending from the first end surface 4220 to the second end surface 4230, wherein the exterior surface 4240 of the cone 4200 is configured to be received in a cavity of the femur such that the first end surface 4220 is flush with or beneath a surface of the resected tibia. The first end surface 4220 is within an axial plane defined by the first end surface 4220 and an outermost edge 4270 (in this embodiment, a perimeter) of the first end surface 4220.

The tibial cone 4200 can be used in a prosthetic system comprising a tibial implant having a body and a stem wherein the stem is positioned within the passageway of the cone 4200 as in Figure 37B. The tibial cone 4200 can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

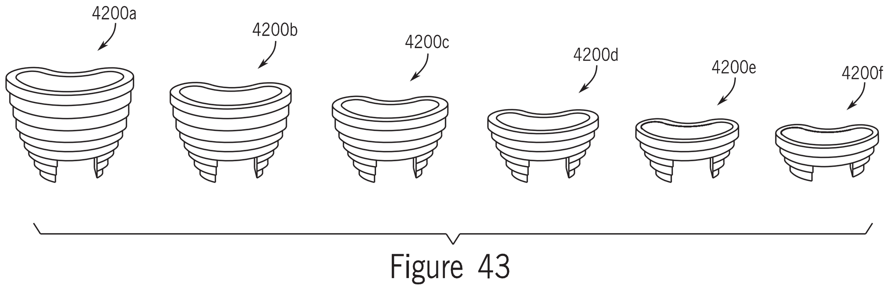

Figure 43 shows top anterior perspective views that correspond to the tibial cone 4200 of Figure 42 wherein the tibial cones 4200a to 4200f decrease in size by removing an incremental step which makes moving up and down sizes intuitive and the instrumentation streamlined.

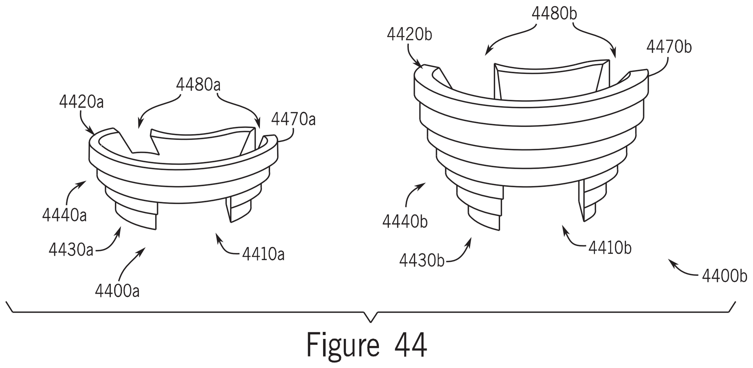

In some sizes of a tibial cone according to the invention, a portion of the distal anterior wall and/or posterior wall may be removed to create notches to accommodate tibial component fins. This can be seen in Figure 44, with notches 4480a of the tibial cone 4400a. Looking at Figure 44, the tibial cone 4400a has a first end surface 4420a, a second end surface 4430a, a stepped exterior surface 4440a extending from the first end surface 4420a to the second end surface 4430a, and an inner surface defining a passageway extending from the first end surface 4420a to the second end surface 4430a, wherein the exterior surface 4440a of the cone 4400a is configured to be received in a cavity of the femur such that the first end surface 4420a is flush with or beneath a surface of the resected tibia. The first end surface 4420a is within an axial plane defined by the first end surface 4420a and an outermost edge 4470a of the first end surface 4420a. The tibial cone 4400a has notches 4480a to accommodate tibial component fins.

Still looking at Figure 44, a tibial cone 4400b (of larger size than tibial cone 4400a) has a first end surface 4420b, a second end surface 4430b, a stepped exterior surface 4440b extending from the first end surface 4420b to the second end surface 4430b, and an inner surface defining a passageway extending from the first end surface 4420b to the second end surface 4430b, wherein the exterior surface 4440b of the cone 4400b is configured to be received in a cavity of the femur such that the first end surface 4420b is flush with or beneath a surface of the resected tibia. The first end surface 4420b is within an axial plane defined by the first end surface 4420b and an outermost edge 4470b of the first end surface 4420b. The tibial cone 4400b has notches 4480b to accommodate tibial component fins.

The tibial cones 4400a and 4400b can be used in a prosthetic system comprising a tibial implant having a body and a stem wherein the stem is positioned within the passageway of the cones 4400a and 4400b as in Figure 37B. The tibial cones 4400a and 4400b can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

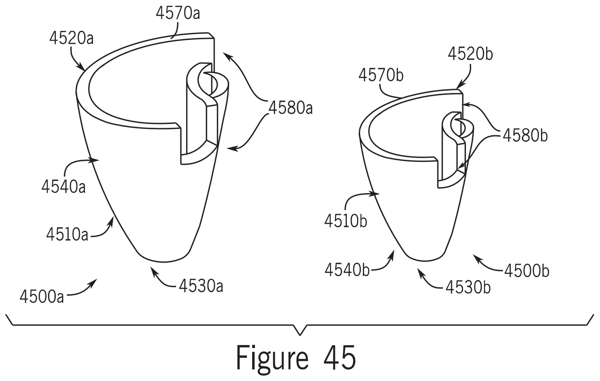

In some sizes of other embodiments of a tibial cone according to the invention, a portion of the distal anterior wall and/or posterior wall may be removed to accommodate tibial component fins. This can be seen in Figure 45, with notches 4580a of the tibial cone 4500a. Looking at Figure 45, the tibial cone 4500a has a first end surface 4520a, a second end surface 4530a, a smooth exterior surface 4540a extending from the first end surface 4420a to the second end surface 4530a, and an inner surface defining a passageway extending from the first end surface 4520a to the second end surface 4530a, wherein the exterior surface 4540a of the cone 4500a is configured to be received in a cavity of the femur such that the first end surface 4520a is flush with or beneath a surface of the resected tibia. The first end surface 4520a is within an axial plane defined by the first end surface 4520a and an outermost edge 4570a of the first end surface 4520a. The tibial cone 4500a has notches 4580a to accommodate tibial component fins.

Still looking at Figure 45, a tibial cone 4500b (of larger size than tibial cone 4500a) has a first end surface 4520b, a second end surface 4530b, a smooth exterior surface 4540b extending from the first end surface 4520b to the second end surface 4530b, and an inner surface defining a passageway extending from the first end surface 4520b to the second end surface 4530b, wherein the exterior surface 4540b of the cone 4500b is configured to be received in a cavity of the femur such that the first end surface 4520b is flush with or beneath a surface of the resected tibia. The first end surface 4520b is within an axial plane defined by the first end surface 4520b and an outermost edge 4570b of the first end surface 4520b. The tibial cone 4500b has notches 4580b to accommodate tibial component fins.

The tibial cones 4500a and 4500b can be used in a prosthetic system comprising a tibial implant having a body and a stem wherein the stem is positioned within the passageway of the cones 4500a and 4500b as in Figure 37B. The tibial cones 4500a and 4500b can be used in a prosthetic system comprising a tibial tray as in Figure 2, and a tibial bearing (as in 130 in Figure 1) in contact with the tibial tray, wherein the tibial bearing has a bearing surface for articulating with the articulating surfaces of a femoral component.

A breadth of sizes greatly broadens the potential for use of cones in revision knee arthroplasty. Current cones have limited sizes with large changes between sizes forcing implants that are too small and do not maximize contact or necessitate additional bone removal to make them fit. The availability of smaller cone sizes in the invention also broadens the market for use of cones which is a premium product with premium pricing which is attractive to manufacturers.

Additionally, the system and implants create a streamlined surgical flow. For the tibia, the distal sizing is confirmed with cylindrical reamers and then broaches for the cones are utilized to ensure maximum support and minimize bone removal. The same is true for the femur after engaging with proximal femoral diaphyseal bone. The system of the invention allows any size cone to be used with any size stem. This greatly facilitates broadening the use of cones in revision cases and expands the market opportunity. For example, a small cone can be used in any size patient providing a better option compared to bone graft, cement, or removing additional bone to make current non-anatomically shaped and sized cones fit.

The cones may be formed from a metal alloy such as titanium alloys (e.g., titanium-6-aluminum-4-vanadium), cobalt-chromium alloys, stainless steel alloys and tantalum alloys; nonresorbable ceramics such as aluminum oxide and zirconia; nonresorbable polymeric materials such as polyethylene; or composite materials such as carbon fiber-reinforced polymers (e.g., polysulfone). In some configurations, the cone is formed from a tantalum based porous material, or it may be made from another metal that is coated with a tantalum-based porous metal or other porous coating.

### F. Cadaveric Validation

Cadaveric validation of the model was performed which clearly demonstrated improved surgical flow as well as performance, feel, and fit compared to current devices on the market. The same instruments may be used for cones and sleeves.

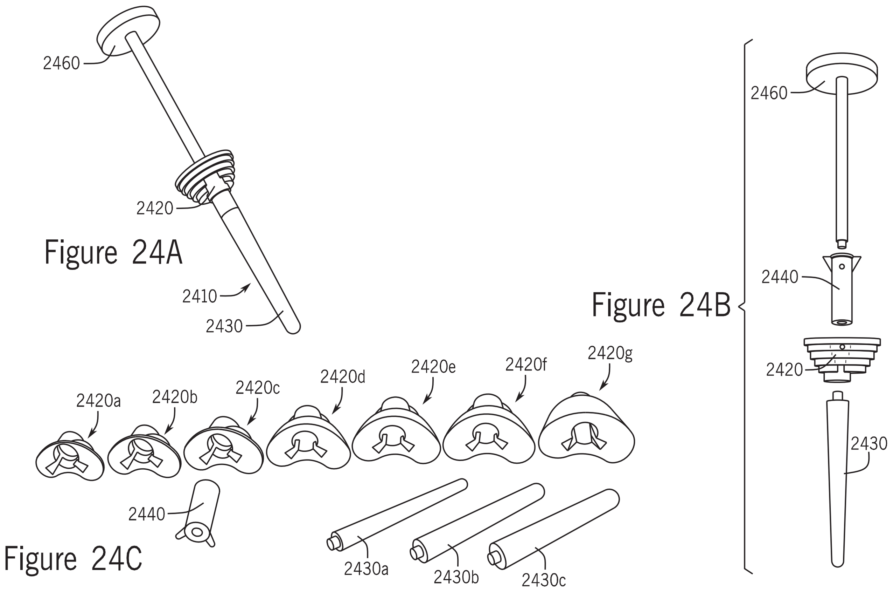

Figures 24A and 24B show a tibial sleeve assembly 2410 including a sleeve 2420, a stem 2430, and an adapter 2440 for connecting the sleeve 2420 and the stem 2430. An insertor 2460 can be used to position the tibial sleeve assembly 2410 in a prepared tibia. Figure 24C shows a range of sizes for the sleeves 2420a to 2420g, and adapter 2440, and a range of sizes for the stem 2430a, 2430b and 2430c. In a tibial sleeve assembly (see Figures 24A and 24B) and close up of the anatomically shaped tibial sleeve assembly (see Figure 24C), one can see how the sleeves are shaped anatomically with an offset of the stem that is based on the true anatomy.

Turning to Figures 25A to 25C, there is shown a femoral sleeve assembly 2510 including a sleeve 2520d, a stem 2530, and an adapter 2540 for connecting the sleeve 2520a and the stem 2530. Figure 25A shows a range of sizes for the sleeves 2520a to 2520f. Figures 25B and 25C show a close up of the anatomically shaped femoral sleeves 2520a to 2520f. One can see how the sleeves are shaped anatomically with the offset and angle of the stem that is based on the true anatomy.

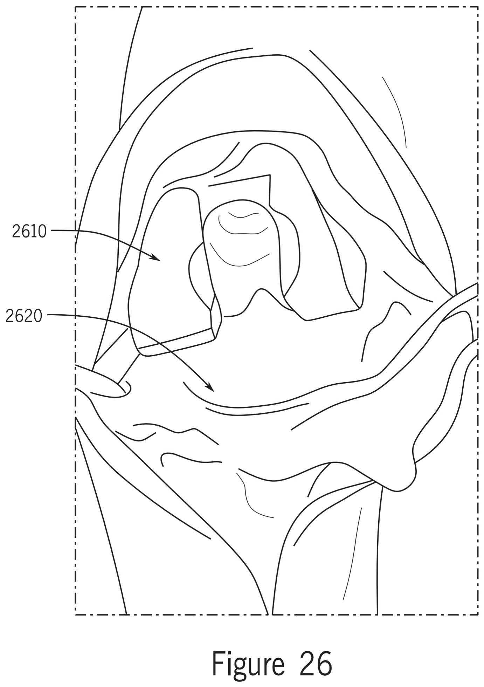

Typical femoral bone defects 2610 and tibial bone defects 2620 in revision knee arthroplasty are shown in Figure 26.

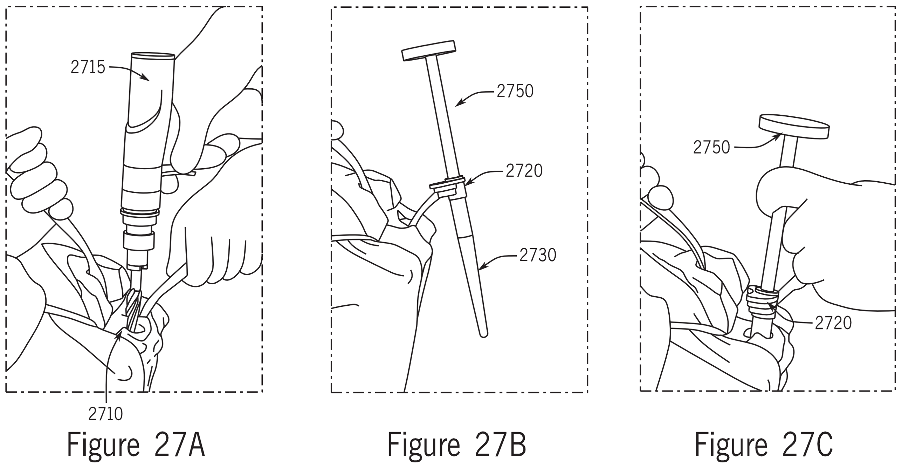

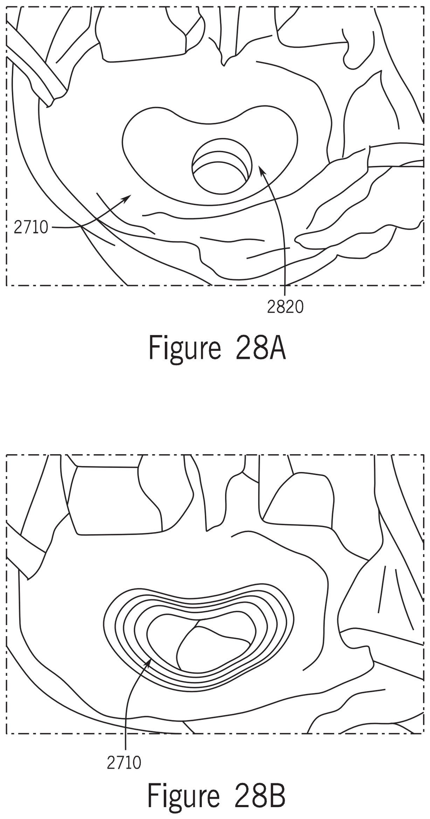

A simplified tibia preparation technique is as follows. Ream distal tibia 2710 with a reamer 2715 to cortical contact (see Figure 27A), tibial broach 2720 and trial stem 2730 (see Figure 27B), and impact broach 2720 and trial stem 2730 with an insertor 2750 (see Figure 27C). The sleeves were designed such that any size sleeve can be used with any size stem. One can see how the sleeve 2820 matches the proximal tibial anatomy and creates an optimized environment for stability and bone ingrowth (see Figures 28A and 28B).

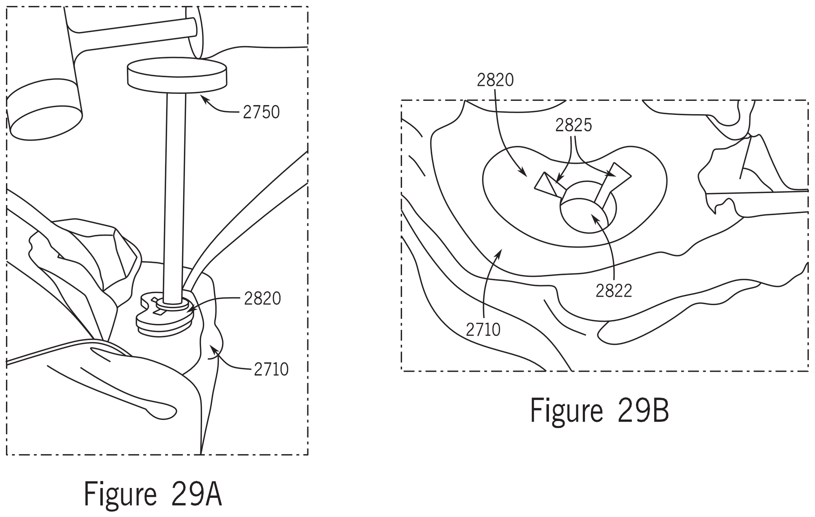

Impaction of the tibial sleeve 2820 and stem 2830 construct is as follows. The slots 2825 extending from the passageway 2822 on the superior aspect of the sleeve 2820 provide the opportunity for additional rotational adjustment of the tibial tray to further improve the tibial coverage an minimize any component overhang. See Figures 29A and 29B.

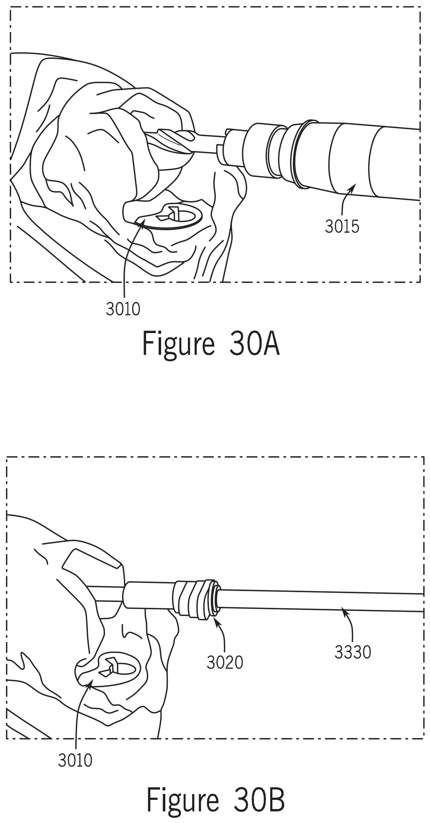

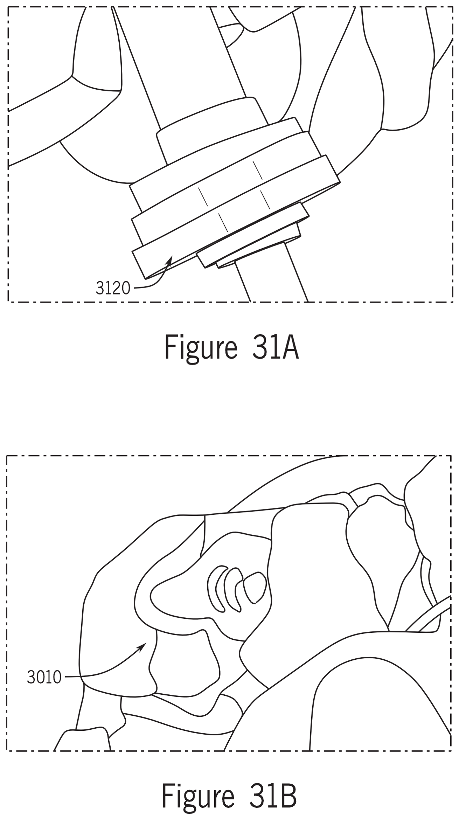

A simplified femoral preparation technique is as follows. Ream femur 3010 to cortical contact with a reamer 3015 (see Figure 30A) and femoral broach 3020 and trial stem 3330 (see Figure 30B). The sleeves were designed such that any size sleeve can be used with any size stem. One can see how the sleeve 3120 matches the distal femoral anatomy 3010 and creates an optimized environment for stability and bone ingrowth (see Figures 31A and 31B).

This disclosure demonstrates the development of a novel methodology that significantly advances the understanding of the proximal tibial and distal femoral anatomy. This methodology facilitated the design of an innovative revision knee arthroplasty system featuring anatomically shaped metaphyseal sleeves. These novel sleeves grow in the medial-lateral and anterior-posterior direction based on the true anatomy significantly improving the contact with the underlying bone resulting in less bone removal, improved fit and fixation. Additionally, the offset of the stems in relation to the sleeves was driven by the methodology allowing for optimized fit, minimizing the risk of iatrogenic fracture, as well as decreasing malalignment of components. The system has an intuitive and streamlined workflow that improves the user experience. The ability to use any size sleeve with any size stem in a bone preserving manner also expands the potential population that can benefit from the use of sleeves at the time of revision knee arthroplasty.

In light of the principles and example embodiments described and illustrated herein, it will be recognized that the example embodiments can be modified in arrangement and detail without departing from such principles. Also, the foregoing discussion has focused on particular embodiments, but other configurations are also contemplated. In particular, even though expressions such as "in one embodiment", "in another embodiment," or the like are used herein, these phrases are meant to generally reference embodiment possibilities, and are not intended to limit the invention to particular embodiment configurations. As used herein, these terms may reference the same or different embodiments that are combinable into other embodiments. As a rule, any embodiment referenced herein is freely combinable with any one or more of the other embodiments referenced herein, and any number of features of different embodiments are combinable with one another, unless indicated otherwise.

Although the invention has been described in considerable detail with reference to certain embodiments, one skilled in the art will appreciate that the present invention can be practiced by other than the described embodiments, which have been presented for purposes of illustration and not of limitation. Therefore, the scope of the appended claims should not be limited to the description of the embodiments contained herein.

## Claims

1. A prosthetic system for providing motion between a first bone and a second bone of a joint, the system comprising:
a support structure having a first end surface, a second end surface, an exterior surface extending from the first end surface to the second end surface, and an inner surface defining a passageway extending from the first end surface to the second end surface, wherein the exterior surface of the support structure is configured to be received in a cavity of the first bone such that the first end surface is flush with or beneath a surface of the first bone,
wherein the first end surface is within an axial plane defined by the first end surface and an outermost edge of the first end surface, and
wherein a longitudinal axis of the passageway of the support structure is offset with respect to a geometric center point of the axial plane.

2. The prosthetic system of claim 1 wherein:
the first bone is the tibia, and
the joint is the knee.

3. The prosthetic system of claim 2 further comprising:
a tibial implant having a body and a stem extending away from the body,
wherein the stem is positioned within the passageway of the support structure.

4. The prosthetic system of claim 3 wherein:
the body of the tibial implant is a tibial tray.

5. The prosthetic system of claim 4 further comprising:
a tibial bearing in contact with the tibial tray, the tibial bearing having a bearing surface for articulating with the articulating surfaces of a femoral component.

6. The prosthetic system of claim 1 wherein:
the first bone is the femur, and
the joint is the knee.

7. The prosthetic system of claim 6 further comprising:
a femoral component having medial and lateral condyles with curved articulating surfaces.

8. The prosthetic system of claim 7 further comprising:
a tibial tray in contact with a tibial bearing having a bearing surface for articulating with the articulating surfaces of the femoral component.

9. The prosthetic system of claim 1 wherein:
the longitudinal axis of the passageway of the support structure is angled at an oblique angle with respect to a normal line to the axial plane.

10. The prosthetic system of claim 9 wherein:
the oblique angle is greater than 0 degrees and less than 10 degrees.

11. The prosthetic system of claim 9 wherein:
the oblique angle is greater than 3 degrees and less than 9 degrees.

12. The prosthetic system of claim 1 wherein:
the longitudinal axis of the passageway of the support structure is an intramedullary axis including a first intersection point and a second intersection point, the first intersection point being defined by a first intersection of an inertial axis of the first bone with a first reference axial plane located at a first distance from an end surface of the first bone before resection, and the second intersection point being defined by a second intersection of the inertial axis of the first bone with a second reference axial plane located at a second distance from the end surface of the first bone before resection, the first distance and the second distance being different.

13. The prosthetic system of claim 12 wherein:
the first distance is between 25 millimeters and 125 millimeters, and
the second distance is between 75 millimeters and 200 millimeters.

14. The prosthetic system of claim 1 further comprising:
a prosthetic implant having a body and a stem extending away from the body,
wherein the stem is positioned within the passageway of the support structure.

15. The prosthetic system of claim 14 wherein:
the support structure is a sleeve, and
the sleeve is attached to the stem of the prosthetic implant.

16. The prosthetic system of claim 14 wherein:
the support structure is a cone.

17. The prosthetic system of claim 1 wherein:
the exterior surface of the support structure is stepped.

18. The prosthetic system of claim 1 wherein:
the exterior surface of the support structure is smooth.

19. The prosthetic system of claim 1 wherein:
the exterior surface of the support structure is roughened.

20. The prosthetic system of claim 1 wherein:
the exterior surface of the support structure comprises a porous ingrowth material.

21. The prosthetic system of claim 1 wherein:
the support structure has a wall between the exterior surface and the inner surface, and
the wall includes one or more notches extending away from the first end surface.

22. The prosthetic system of claim 21 wherein:
the wall is an anterior wall.

23. The prosthetic system of claim 21 wherein:
the wall is a posterior wall.

24. The prosthetic system of claim 1 wherein:
the support structure has a wall between the exterior surface and the inner surface, and
the wall includes one or more notches extending away from the second end surface.

25. The prosthetic system of claim 24 wherein:
the wall is an anterior wall.

26. The prosthetic system of claim 24 wherein:
the wall is a posterior wall.

27. The prosthetic system of claim 1 wherein:
the support structure has a wall between the exterior surface and the inner surface, and
an anterior section of the wall has reduced thickness compared to another section of the wall adjacent to the anterior section of the wall.

28. The prosthetic system of claim 1 wherein:
the outermost edge of the first end surface is a perimeter of the first end surface.

29. The prosthetic system of claim 1 wherein:
the first end surface includes one or more slots extending from the passageway, and
each slot is dimensioned to receive a stabilization arm of a stem of a prosthetic implant.

30. A kit for a prosthetic system for providing motion between a first bone and a second bone of a joint, the kit comprising:
(i) a first support structure having a first end surface, a second end surface, an exterior surface extending from the first end surface to the second end surface, and an inner surface defining a passageway extending from the first end surface to the second end surface, wherein the exterior surface of the first support structure is configured to be received in a cavity of the first bone such that the first end surface is flush with or beneath a surface of the first bone, wherein the first end surface is within an axial plane defined by the first end surface and an outermost edge of the first end surface, and wherein a longitudinal axis of the passageway of the first support structure is offset with respect to a geometric center point of the axial plane; and
(ii) one or more additional support structures, each additional support structure having an end surface within an additional axial plane defined by the end surface of each additional support structure and a border of the end surface of each additional support structure, wherein each longitudinal axis of a passageway extending through each additional support structure is offset with respect to a geometric center point of the additional axial plane.

31. The kit of claim 30 wherein:
the first bone is the tibia,
the joint is the knee, and
the first support structure and each additional support structure are configured to be received in a cavity of the tibia.

32. The kit of claim 30 wherein:
the first bone is the femur,
the joint is the knee, and
the first support structure and each additional support structure are configured to be received in a cavity of the femur.

33. The kit of claim 30 wherein:
the longitudinal axis of the passageway of the first support structure is angled at an oblique angle with respect to a normal line to the axial plane.

34. The kit of claim 33 wherein:
the oblique angle is greater than 0 degrees and less than 10 degrees.

35. The kit of claim 33 wherein:
the oblique angle is greater than 3 degrees and less than 9 degrees.

36. The kit of claim 32 wherein:
the longitudinal axis of the passageway of the first support structure is an intramedullary axis including a first intersection point and a second intersection point, the first intersection point being defined by a first intersection of an inertial axis of the first bone with a first reference axial plane located at a first distance from an end surface of the first bone before resection, and the second intersection point being defined by a second intersection of the inertial axis of the first bone with a second reference axial plane located at a second distance from the end surface of the first bone before resection, the first distance and the second distance being different.

37. The kit of claim 30 wherein:
the first support structure and each additional support structure are a sleeve, and
each sleeve is dimensioned to be attached to a stem of a prosthetic implant.

38. The kit of claim 30 wherein:
the first support structure and each additional support structure are a cone.

39. The kit of claim 30 wherein:
the exterior surface of the first support structure is stepped.

40. The kit of claim 30 wherein:
the exterior surface of the first support structure is smooth.

41. The kit of claim 30 wherein:
the exterior surface of the support structure is roughened.

42. The kit of claim 30 wherein:
the exterior surface of the support structure comprises a porous ingrowth material.

43. The kit of claim 30 wherein:
the first support structure has a wall between the exterior surface and the inner surface, and
the wall includes one or more notches extending away from the first end surface.

44. The kit of claim 43 wherein:
the wall is an anterior wall.

45. The kit of claim 43 wherein:
the wall is a posterior wall.

46. The kit of claim 30 wherein:
the first support structure has a wall between the exterior surface and the inner surface, and
the wall includes one or more notches extending away from the second end surface.

47. The kit of claim 46 wherein:
the wall is an anterior wall.

48. The kit of claim 46 wherein:
the wall is a posterior wall.

49. The kit of claim 30 wherein:
the first support structure has a wall between the exterior surface and the inner surface, and
an anterior section of the wall has reduced thickness compared to another section of the wall adjacent to the anterior section of the wall.

50. The kit of claim 30 wherein:
the outermost edge of the first end surface is a perimeter of the first end surface.

51. The kit of claim 30 wherein:
the first end surface includes one or more slots extending from the passageway, and
each slot is dimensioned to receive a stabilization arm of a stem of a prosthetic implant.
